(19) Europäisches Patentamt
European Patent Office
Office européen des brevets

(11) **EP 4 339 186 A1**

(12) **EUROPEAN PATENT APPLICATION**
published in accordance with Art. 153(4) EPC

(43) Date of publication:
**20.03.2024 Bulletin 2024/12**

(21) Application number: **22842329.9**

(22) Date of filing: **28.06.2022**

(51) International Patent Classification (IPC):
***C07C 219/06*** *(2006.01)* ***C07C 219/20*** *(2006.01)*
***A01N 37/44*** *(2006.01)*

(52) Cooperative Patent Classification (CPC):
**A01N 37/44; C07C 219/06; C07C 219/20**

(86) International application number:
**PCT/KR2022/009225**

(87) International publication number:
**WO 2023/287067 (19.01.2023 Gazette 2023/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.07.2021 KR 20210093703**
**27.06.2022 KR 20220078380**

(71) Applicant: **LG Chem, Ltd.**
**Yeongdeungpo-gu**
**Seoul 07336 (KR)**

(72) Inventors:
- **BAEK, Leehyeon**
  **Daejeon 34122 (KR)**
- **LEE, Ji Seok**
  **Daejeon 34122 (KR)**
- **CHOI, Hyungsam**
  **Daejeon 34122 (KR)**
- **JUNG, Seonjung**
  **Daejeon 34122 (KR)**
- **KANG, Soonhee**
  **Daejeon 34122 (KR)**
- **YUN, Haesung**
  **Daejeon 34122 (KR)**
- **HUR, Yoon Hyung**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **ANTIBACTERIAL COMPOUND**

(57) Provided is a novel compound, more particularly, a novel compound exhibiting an excellent antibacterial property while having a polymerizable functional group to prepare an antibacterial polymer.

【FIG. 3】

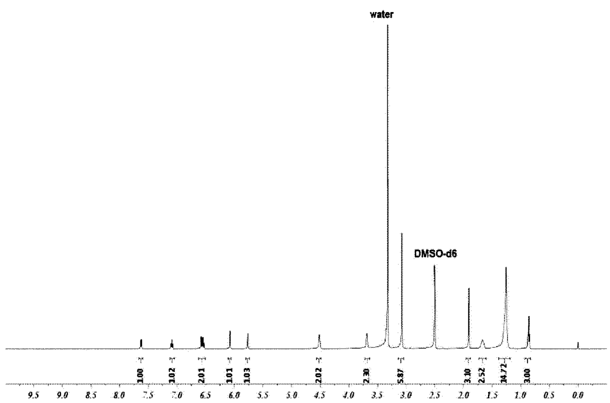


EP 4 339 186 A1

## Description

[Technical Field]

Cross-reference to Related Application

The present application is based on, and claims priority from, Korean Patent Application Nos. 10-2021-0093703, and 10-2022-0078380, filed on July 16, 2021, and June 27, 2022, respectively, the disclosures of which are hereby incorporated by reference herein in their entirety.

The present disclosure relates to a novel compound exhibiting an antibacterial property.

[Background Art]

Recently, with the diversification of life, improvement of living standards, and change and improvement of consciousness, interest in improving hygiene and comfort in an individual's living environment is increasing. Accordingly, studies have been conducted on microorganisms that threaten them, but there are many types of microorganisms in the daily living environment, and they are widespread in nature, causing serious problems.

In particular, microorganisms such as bacteria, fungi, etc. may inhabit various environments, such as dietary life, residential environments, clothing, industrial products, etc. However, since bacteria may cause various inflammations or food poisoning, and fungi may produce not only odors, but also various skin diseases, respiratory diseases, allergies, atopic dermatitis, etc., they are problematic. In addition, microorganisms living on the surface of electronic products and household goods may cause deterioration of product performance.

Accordingly, in order to prevent human damage caused by these microorganisms, various antibacterial substances have been developed to inhibit the growth of microorganisms or to kill microorganisms.

Specifically, antibacterial agents previously developed may be broadly divided into inorganic antibacterial agents and organic antibacterial agents. Inorganic antibacterial agents are antibacterial agents containing a metal such as silver, copper, etc., and have an advantage of maintaining the antibacterial property even under high temperature conditions due to excellent thermal stability. However, there are problems in that they are expensive and there is a possibility of discoloration due to the metal ions contained after processing. In addition, organic antibacterial agents have advantages in that they are cheaper than inorganic antibacterial agents and exhibit excellent antibacterial effects even in a small amount. However, organic antibacterial agents have a problem in that antibacterial durability is poor because there is a possibility of leaching after being applied to articles.

Moreover, although organic antibacterial agent may secure stability of products in terms of inhibiting microbial growth and killing microorganisms, they are toxic to cause irritation to the user's skin.

For this reason, it has been discussed to introduce an antibacterial substance in the form of a polymer into an article in order to prevent a decrease in the antibacterial property and safety problems due to the leaching of the antibacterial substance. Accordingly, there is a need for an antibacterial monomer which has a polymerizable functional group to synthesize an antibacterial polymer while having excellent antibacterial property of the compound itself.

[Prior Art Document]

[Patent Document]

(Patent Document 0001) Korean Patent No. 10-0601393

[Disclosure]

[Technical Problem]

There is provided a novel compound having an excellent bacterial growth-inhibitory effect.

There is also provided an antibacterial agent including the compound.

[Technical Solution]

To achieve the above objects,

there is provided a compound represented by the following Chemical Formula 1:

[Chemical Formula 1]

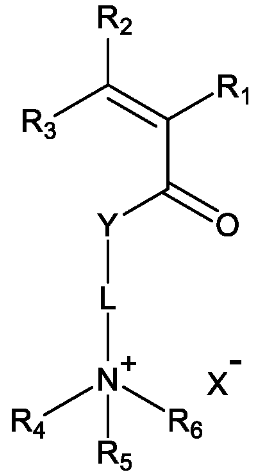

in Chemical Formula 1,
Y is O, S, or $N(R_7)$,
L is a single bond, alkylene having 1 to 10 carbon atoms, or arylene having 6 to 60 carbon atoms,
$R_1$ to $R_3$ are each independently hydrogen or methyl,
$R_4$ to $R_7$ are each independently hydrogen, substituted or unsubstituted alkyl having 1 to 20 carbon atoms, or substituted or unsubstituted aryl having 6 to 60 carbon atoms, and
$X^-$ is a conjugate base of an aromatic acid having 6 to 20 carbon atoms, which is substituted with one or more hydroxyl groups,
wherein the conjugate base of the aromatic acid may be further substituted with one or more substituents selected from the group consisting of halogen, alkyl having 1 to 4 carbon atoms, haloalkyl having 1 to 4 carbon atoms, and alkoxy having 1 to 4 carbon atoms.

There is also provided an antibacterial agent including the compound represented by Chemical Formula 1.

[Effect of the Invention]

A compound according to the present disclosure is advantageous in that it has an excellent bacterial growth-inhibitory effect and has a polymerizable functional group to prepare a polymer exhibiting an antibacterial property.

[Brief Description of Drawings]

FIG. 1 shows an MS spectrum of Compound A;
FIG. 2 shows an $^1H$ NMR spectrum of Compound A;
FIG. 3 shows an $^1H$ NMR spectrum of Compound 1; and
FIG. 4 shows a mass spectrum of Compound 1.

[Detailed Description]

In the present invention, the terms "the first", "the second", and the like are used to describe a variety of components, and these terms are merely employed to differentiate a certain component from other components.
Further, the terms used in this description are just for explaining exemplary embodiments and it is not intended to restrict the present invention. The singular expression may include the plural expression unless it is differently expressed contextually. It must be understood that the term "include", "equip", or "have" in the present description is only used for designating the existence of characteristics taken effect, numbers, steps, components, or combinations thereof, and do not exclude the existence or the possibility of addition of one or more different characteristics, numbers, steps, components or combinations thereof beforehand.
Further, in the present invention, when a layer or an element is mentioned to be formed "on" or "above" layers

or elements, it means that each layer or element is directly formed on the layers or elements, or other layers or elements may be formed between the layers, subjects, or substrates.

The present invention may be variously modified and have various forms, and specific exemplary embodiments are exemplified and explained in detail in the following description. However, it is not intended to limit the present invention to the specific exemplary embodiments and it must be understood that the present invention includes every modifications, equivalents, or replacements included in the spirit and technical scope of the present invention.

Further, the terms used in this description are just for explaining exemplary embodiments and it is not intended to restrict the present invention. The singular expression used herein may include the plural expression unless it is differently expressed contextually.

Meanwhile, as used herein, the term "(meth)acrylate" includes both acrylate and methacrylate.

In addition, in the present specification, the alkyl group may be straight or branched, and its number of carbon atoms is not particularly limited, but preferably 1 to 20. According to one embodiment, the number of carbon atoms of the alkyl group is 1 to 16. According to one embodiment, the number of carbon atoms of the alkyl group is 1 to 12. According to one embodiment, the number of carbon atoms of the alkyl group is 8 to 12. Specific examples of the alkyl group may include methyl, ethyl, propyl, n-propyl, isopropyl, butyl, n-butyl, isobutyl, tert-butyl, sec-butyl, 1-methyl-butyl, 1-ethylbutyl, pentyl, n-pentyl, isopentyl, neopentyl, tert-pentyl, 1-ethyl-propyl, 1,1-dimethylpropyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, 2-methylhexyl, 3-methylhexyl, 4-methylhexyl, 5-methylhexyl, octyl, n-octyl, tert-octyl, 1-methylheptyl, 2-ethylhexyl, 2,4,4-trimethyl-1-pentyl, 2,4,4-trimethyl-2-pentyl, 2-propylpentyl, n-nonyl, 2,2-dimethylheptyl, etc., but are not limited thereto. Further, in the present specification, the above description of the alkyl group may also be applied to alkylene, except that the alkylene is a divalent group.

Further, in the present specification, the aryl group is not particularly limited, but may preferably have 6 to 60 carbon atoms, and may be a monocyclic aryl group or a polycyclic aryl group. According to one embodiment, the number of carbon atoms of the aryl group is 6 to 20. According to one embodiment, the number of carbon atoms of the aryl group is 6 to 10. The monocyclic aryl group may include a phenyl group, a biphenyl group, a terphenyl group, etc., but is not limited thereto. The polycyclic aryl group may include a naphthyl group, an anthracenyl group, a phenanthryl group, a pyrenyl group, a perylenyl group, a chrysenyl group, a fluorenyl group, etc., but is not limited thereto. Further, in the present specification, the above description of the aryl group may also be applied to arylene, except that the arylene is a divalent group.

Further, in the present specification, the term "substituted or unsubstituted" may be understood as the meaning of "being unsubstituted or substituted with one or more, for example, 1 to 5 substituents selected from the group consisting of deuterium, halogen, cyano, alkyl having 1 to 10 carbon atoms, alkoxy having 1 to 10 carbon atoms, and aryl having 6 to 20 carbon atoms.

Further, in the present specification, the alkoxy group is a substituent in which an oxygen atom is bound to a connection portion with another atom in the alkyl group, and the above description of the alkyl group may also be applied to the alkyl group.

In order to bring antibacterial property to household chemical products generally used in daily living space, such as homes, offices, multiuse facilities, etc., antibacterial coating capable of preventing and/or killing growth of microorganisms such as bacteria is performed on the surface of the household chemical products. At this time, antibacterial agents contained in the antibacterial coating damage the cell membrane or cell wall of microorganisms or induce denaturation of their proteins, thereby inhibiting the growth of microorganisms, leading to preventing reproduction of microorganisms and/or killing microorganisms.

Further, there are more than 5,000 species of bacteria that have been identified. Specifically, bacteria have diverse cell morphologies such as spheres, rods, spirals, etc., and their demand for oxygen is also different from each other, and thus they are divided into aerobic bacteria, facultative bacteria, and anaerobic bacteria. Therefore, it is usually not easy for one type of antibacterial agent to have physical/chemical mechanisms whereby cell membranes/cell walls of various bacteria are damaged or proteins thereof are denatured.

In addition, there have been a problem that the antibacterial agent used in the article was leached over time, and there has been a concern that a user's health may be threatened by the antibacterial substance, when the user is exposed to the leached antibacterial agent. Accordingly, it has been discussed to introduce the antibacterial agent in the form of a polymer rather than a single compound, in order to prevent degradation of antibacterial property and safety problems due to the leaching of the antibacterial substance.

Accordingly, the present inventors found that when a compound has a structure of a salt compound, in which a quaternary ammonium cation having a polymerizable functional group and an anion having a specific structure are bound, the compound itself may exhibit antibacterial property against at least one of Gram-positive bacteria and Gram-negative bacteria, more specifically, all of Gram-positive bacteria and Gram-negative bacteria, while enabling synthesis of an antibacterial polymer such as a homopolymer or a copolymer due to the polymerizable functional group introduced into the molecule, thereby completing the present disclosure.

In particular, the anion included in the compound is not a halogen anion, but a conjugate base of an aromatic acid having 6 to 20 carbon atoms, which is substituted with one or more hydroxyl groups (-OH). More specifically, the anion has an aromatic ring compound structure, in which one or more hydroxyl groups (-OH) and one or more carboxylate group (-COO-) are substituted. In the case of having such a structure, the hydroxyl group (-OH) contained in the molecule interacts with the surface of the bacterial cell to decompose the cell membrane and to coagulate the contents, thereby further improving the antibacterial property of the compound. In addition, a stable salt may be formed due to the carboxylate group (COO-) contained in the molecule.

In addition, as used herein, the meaning of "exhibiting an antibacterial property against a specific bacterium" is that a compound (bacteriostatic substance) to be tested whether or not it has an antibacterial property is added to a culture medium of the test bacterium, and after culturing, the number of bacteria is significantly reduced, as compared to a reference without the bacteriostatic substance, and specifically, it means that an antibacterial rate (%) calculated by the following Equation 1 according to evaluation of the antibacterial property to be described later is 70% or more.

[Equation 1]

$$\text{Antibacterial rate (\%)} = (1 - A_s/A_0) \times 100$$

in the equation,

$A_s$($A_{sample}$) represents absorbance of the culture medium containing the bacteriostatic substance at a wavelength of 600 nm, and

$A_0$($A_{reference}$) represents absorbance of the pure culture medium without the bacteriostatic substance at a wavelength of 600 nm.

More preferably, "exhibiting an antibacterial property against a specific bacterium" means that the antibacterial rate (%) calculated according to Equation 1 is 70% or more, 70.6% or more, 75.5% or more, 80% or more, 90% or more, 95% or more, 95.3% or more, 95.8% or more, 96% or more, 97% or more, 97.3% or more, 98% or more, 98.1 % or more, and 100% or less.

Further, the Gram-positive bacteria collectively refer to bacteria that are stained purple when stained by the Gram staining method. The cell wall of Gram-positive bacteria consists of several layers of peptidoglycan. After staining with a basic dye such as crystal violet, even when the Gram-positive bacteria are treated with ethanol, they are not decolorized and remain colored purple. Bacteria categorized as the Gram-positive bacteria include *Enterococcus faecalis, Staphylococcus aureus, Streptococcus pneumoniae, Enterococcus faecium, Lactobacillus lactis,* etc.

Further, the Gram-negative bacteria collectively refer to bacteria that are stained red when stained by the Gram staining method, and have outer membrane consisting of lipopolysaccharides, lipoproteins, and other complex polymer materials instead of the cell wall having a relatively small amount of peptidoglycan, as compared to the Gram-positive bacteria. Thus, after staining with a basic dye such as crystal violet, even when the Gram-negative bacteria are treated with ethanol, they are decolorized, and when they are counter-stained with a red dye such as safranin, they appear red. Bacteria categorized as the Gram-negative bacteria include *Proteus mirabilis, Escherichia coli, Salmonella typhi, Pseudomonas aeruginosa, Vibrio cholerae,* etc.

Therefore, since the Gram-positive bacteria and Gram-negative bacteria may cause various diseases upon contacting, and may also cause secondary infection in critically ill patients with weak immunity, it is preferable that a single antibacterial agent is used to exhibit the antibacterial property against both Gram-positive bacteria and Gram-negative bacteria.

Meanwhile, the compound according to one embodiment may exhibit the antibacterial property against at least one of Gram-positive bacteria and Gram-negative bacteria due to the cation and the anion of the quaternary ammonium salt. Specifically, the ammonium cation of the quaternary ammonium salt is electrostatically adsorbed onto the cell wall of Gram-positive bacteria or Gram-negative bacteria, and then interaction with the alkyl group of the hydrophobic quaternary ammonium salt occurs, and as a result, the cell surface structure of the bacteria is destroyed to inhibit growth of the bacteria. Furthermore, the hydroxyl group (-OH) of the anion of the quaternary ammonium salt interacts with the surface of the bacterial cell to decompose the cell membrane and coagulate the contents, thereby further inhibiting growth of the bacteria.

Hereinafter, a compound and an antibacterial agent including the same will be described in more detail according to specific embodiments of the present invention.

## Compound

A compound of one embodiment is represented by the following Chemical Formula 1:

[Chemical Formula 1]

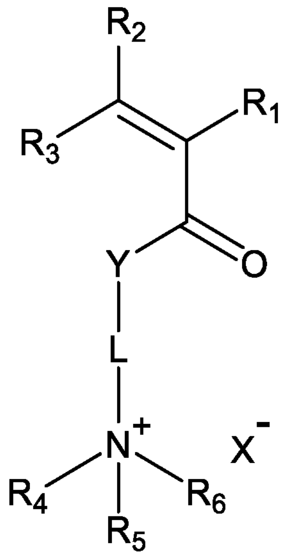

in Chemical Formula 1,

Y is O, S, or $N(R_7)$,
L is a single bond, alkylene having 1 to 10 carbon atoms, or arylene having 6 to 60 carbon atoms,
$R_1$ to $R_3$ are each independently hydrogen or methyl,
$R_4$ to $R_7$ are each independently hydrogen, substituted or unsubstituted alkyl having 1 to 20 carbon atoms, or substituted or unsubstituted aryl having 6 to 60 carbon atoms, and
$X^-$ is a conjugate base of an aromatic acid having 6 to 20 carbon atoms, which is substituted with one or more hydroxyl groups,
wherein the conjugate base of the aromatic acid may be unsubstituted or substituted with one or more substituents selected from the group consisting of halogen, alkyl having 1 to 4 carbon atoms, haloalkyl having 1 to 4 carbon atoms, and alkoxy having 1 to 4 carbon atoms, in addition to one or more hydroxyl groups.

Here, the conjugate base of the aromatic acid may be further substituted with one or more substituents selected from the group consisting of halogen, alkyl having 1 to 4 carbon atoms, haloalkyl having 1 to 4 carbon atoms, and alkoxy having 1 to 4 carbon atoms.

In other words, the conjugate base of the aromatic acid may be unsubstituted or substituted with one or more substituents, for example, 1 to 5 substituents selected from the group consisting of halogen, alkyl having 1 to 4 carbon atoms, haloalkyl having 1 to 4 carbon atoms, and alkoxy having 1 to 4 carbon atoms, in addition to one or more hydroxyl groups.

In Chemical Formula 1, Y may be O, S, or NH. Preferably, Y may be O.

Further, $R_1$ may be hydrogen or methyl, and $R_2$ and $R_3$ may be hydrogen.

Further, one of the three terminal groups, $R_4$, $R_5$ and $R_6$ substituents substituted in the quaternary ammonium cation of the first repeating unit may be alkyl having 5 to 20 carbon atoms. More specifically, one of $R_4$ to $R_6$ may be a linear, i.e., straight chain alkyl having 5 to 20 carbon atoms. In this regard, when all of $R_4$, $R_5$ and $R_6$ substituents are alkyl having less than 5 carbon atoms, there is a problem in that the antibacterial property may not be exhibited, and when any one of $R_4$, $R_5$ and $R_6$ substituents is alkyl having more than 20 carbon atoms, the starting material for preparing the copolymer is not dissolved in the solvent, and thus the synthesis itself is impossible.

More specifically, one of $R_4$ to $R_6$ may be alkyl having 5 to 20 carbon atoms, and the others may be each independently alkyl having 1 to 4 carbon atoms.

For example, one of $R_4$ to $R_6$ may be alkyl having 5 to 20 carbon atoms, and the others may be each independently methyl or ethyl. Preferably, $R_5$ may be alkyl having 5 to 20 carbon atoms, and $R_4$ and $R_6$ may be each independently methyl or ethyl.

More specifically, $R_1$ may be methyl, $R_2$ and $R_3$ may be hydrogen, one of $R_4$ to $R_6$ may be alkyl having 5 to 20 carbon atoms, and the others may be each independently methyl or ethyl; or
all of $R_1$ to $R_3$ may be hydrogen, one of $R_4$ to $R_6$ may be alkyl having 5 to 20 carbon atoms, and the others may be each independently methyl or ethyl.

Further, for example, one of $R_4$ to $R_6$ may be alkyl having 6 to 16 carbon atoms, and the others may be each independently methyl or ethyl. Preferably, $R_5$ may be alkyl having 6 to 16 carbon atoms, and $R_4$ and $R_6$ may be each independently methyl or ethyl.

Further, for example, one of $R_4$ to $R_6$ may be alkyl having 8 to 12 carbon atoms, and the others may be each independently methyl or ethyl. Preferably, $R_5$ may be alkyl having 8 to 12 carbon atoms, and $R_4$ and $R_6$ may be each independently methyl or ethyl.

In addition, among $R_4$, $R_5$ and $R_6$ substituents, two substituents other than alkyl having 5 to 20 carbon atoms may be the same as each other.

Preferably, in Chemical Formula 1, one of $R_4$, $R_5$ and $R_6$ may have 6 or more, 7 or more, or 8 or more carbon atoms, and 20 or less, 18 or less, 16 or less, 14 or less, or 12 or less carbon atoms.

For example, $R_1$ may be methyl, $R_2$ and $R_3$ may be hydrogen, $R_4$ may be alkyl having 6 to 16 carbon atoms, and $R_5$ and $R_6$ may be each independently methyl or ethyl. The antibacterial copolymer including the first repeating unit of the structure may exhibit excellent antibacterial property against at least one of Gram-positive bacteria and Gram-positive bacteria, more specifically, all of Gram-positive bacteria and Gram-negative bacteria.

Further, $R_7$ may be hydrogen, alkyl having 1 to 4 carbon atoms, or aryl having 6 to 20 carbon atoms. For example, $R_7$ may be hydrogen, methyl, or phenyl.

Meanwhile, in Chemical Formula 1, the compound includes a conjugate base of an aromatic acid having 6 to 20 carbon atoms, which is substituted with one or more hydroxyl groups (-OH), more specifically, an aromatic acid having 6 to 20 carbon atoms, which is substituted with 1 to 3 hydroxyl groups as a counter ion of the quaternary ammonium cation moiety.

As used herein, the "aromatic acid" is a kind of aromatic compound containing both an aromatic ring and an organic acid functional group, and specifically, refers to a compound in which one or more carboxyl groups (-COOH) are substituted on an aromatic ring having 6 to 20 carbon atoms. Therefore, the "conjugate base of an aromatic acid" may mean a compound in which one or more carboxylate groups ($-COO^-$) are substituted on an aromatic ring having 6 to 20 carbon atoms, wherein a hydrogen ion ($H^+$) is donated from the carboxyl group.

In other words, $X^-$ is an aromatic ring compound having 6 to 20 carbon atoms, in which one or more hydroxyl groups and one or more carboxylate groups are substituted. When $X^-$ is such a compound, the anion substitution reaction is advantageous, and thus preparation may be easy, and the antibacterial activity of the compound may also be further improved.

In this regard, the conjugate base of an aromatic acid may be further substituted with one or more, more specifically, 1 to 3 substituents selected from the group consisting of halogen, alkyl having 1 to 4 carbon atoms, haloalkyl having 1 to 4 carbon atoms, and alkoxy having 1 to 10 carbon atoms, in addition to the hydroxyl groups.

For example, $X^-$ may be represented by the following Chemical Formula 2:

[Chemical Formula 2]

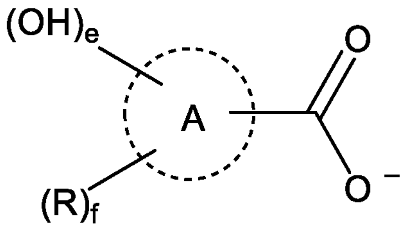

in Chemical Formula 2,

A is a benzene ring, or a naphthalene ring,
R is fluoro, bromo, chloro, iodo, methyl, ethyl, propyl, isopropyl, butyl, isobutyl, sec-butyl, tert-butyl, trifluoromethyl, methoxy, or ethoxy,
e is 1, 2, or 3, and
f is an integer of 0 to 5,
wherein when f is 2 or more, two or more R's are the same as or different from each other.

Specifically, in Chemical Formula 2,

R may be fluoro, bromo, chloro, methyl, trifluoromethyl, or methoxy,
e may be 1, 2, or 3, and
f may be 0, 1, 2, or 3.

At this time, e+f may be 1, 2, or 3.

More specifically, $X^-$ may be represented by any one of the following Chemical Formulae 2-1 to 2-6:

[Chemical Formula 2-1]

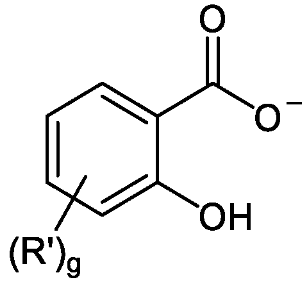

[Chemical Formula 2-2]

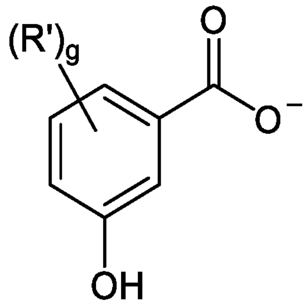

[Chemical Formula 2-3]

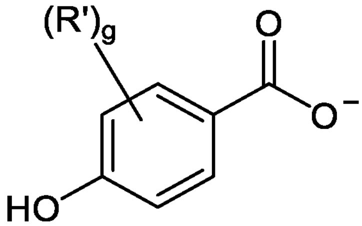

[Chemical Formula 2-4]

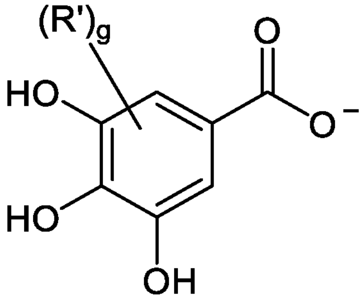

[Chemical Formula 2-5]

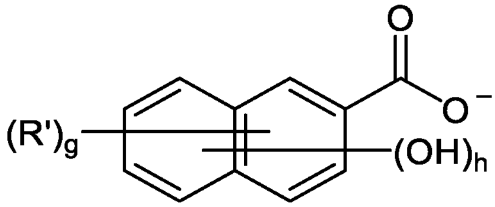

[Chemical Formula 2-6]

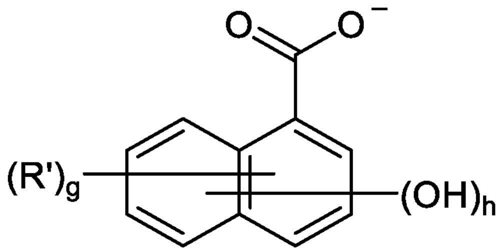

in Chemical Formulae 2-1 to 2-6,

R' is fluoro, bromo, chloro, methyl, trifluoromethyl, or methoxy,
g is 0, 1, or 2, and
wherein when g is 2, two R's are the same as or different from each other, h is 1 or 2.

At this time, g+h may be 1, 2, or 3.

For example, $X^-$ is any one selected from the group consisting of:

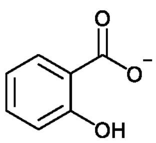 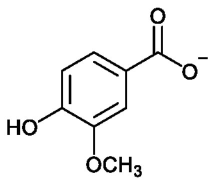 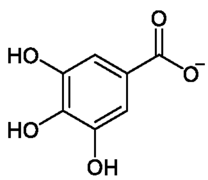 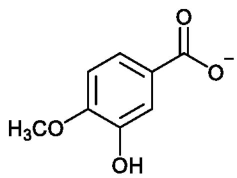

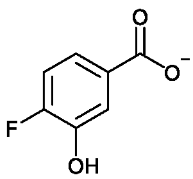 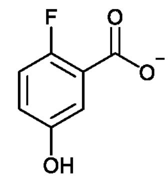 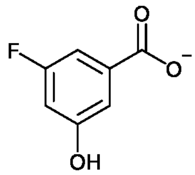 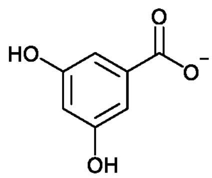

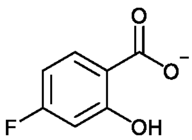 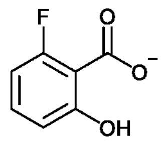 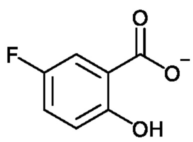 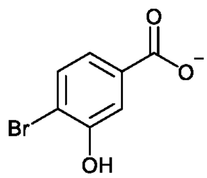

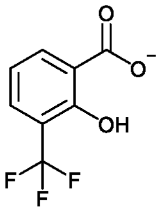 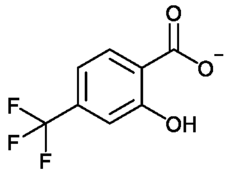 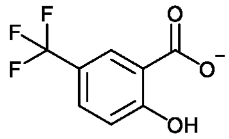 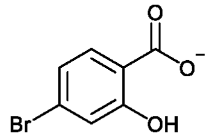

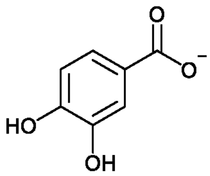 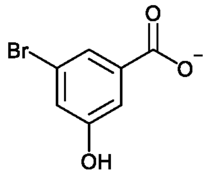 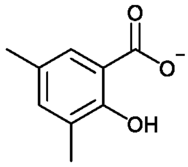 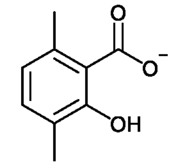

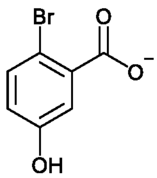 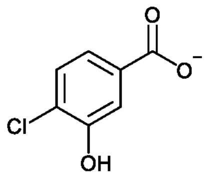 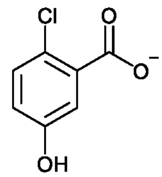 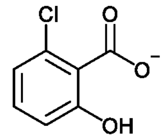

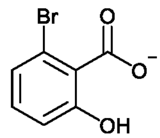 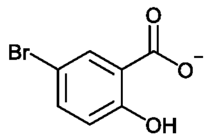 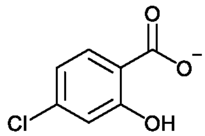 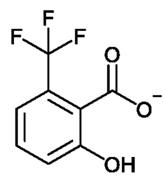

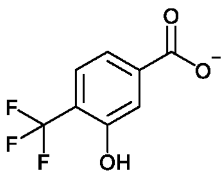 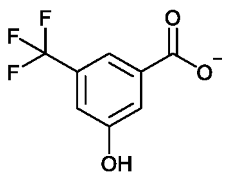 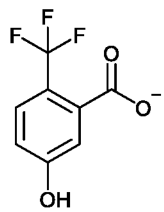 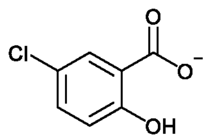

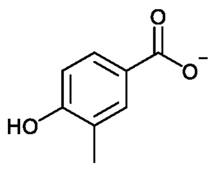 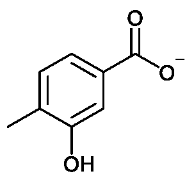 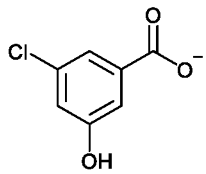

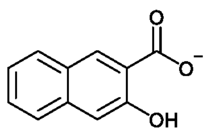 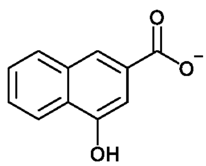 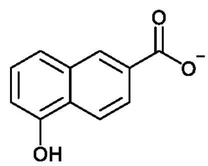 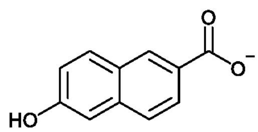

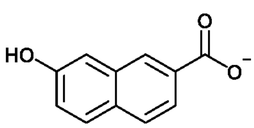 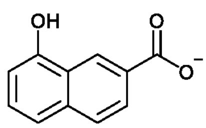 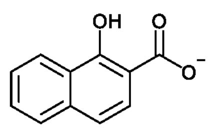 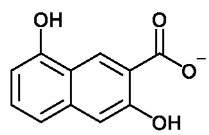

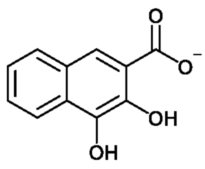
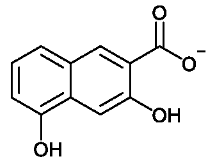
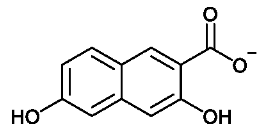
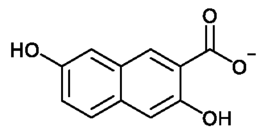

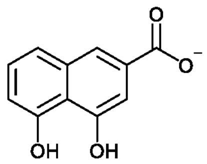
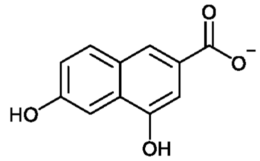
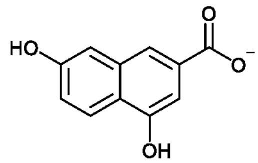
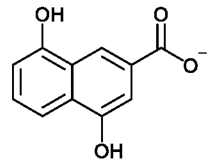

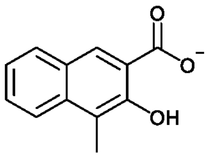
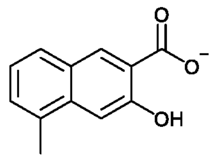
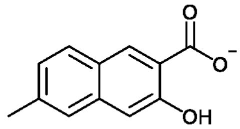
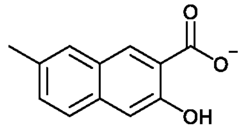

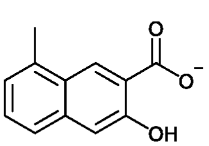
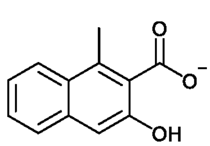
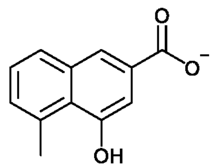
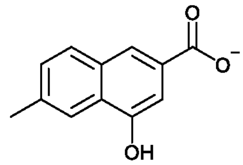

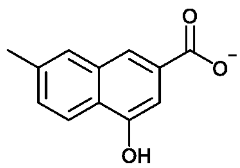
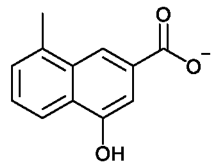
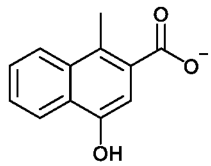
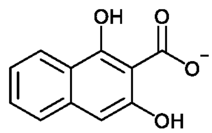

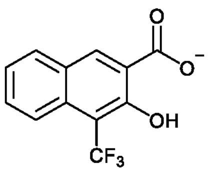
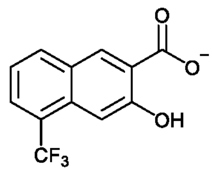
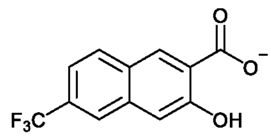
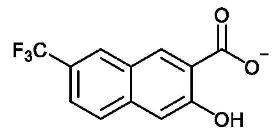

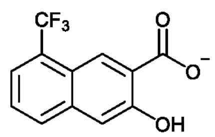
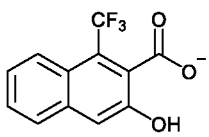
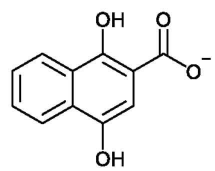

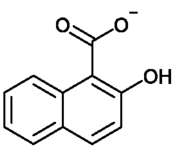
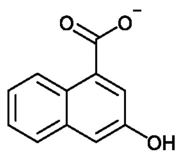
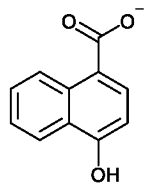
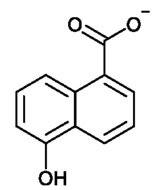

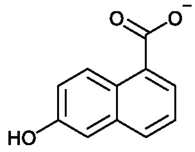 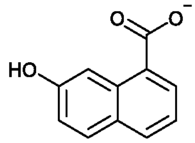 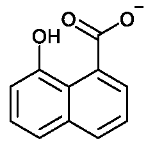

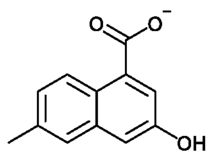 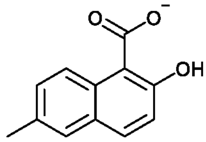 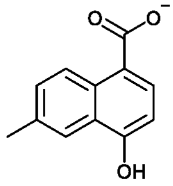 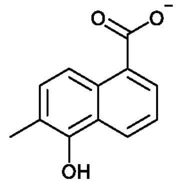

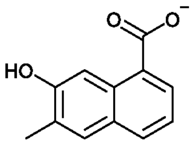 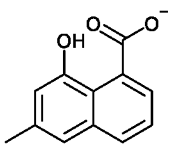 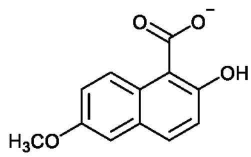 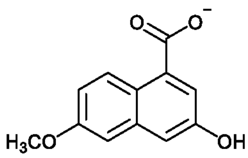

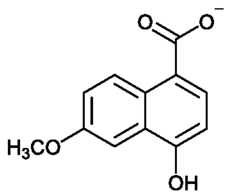 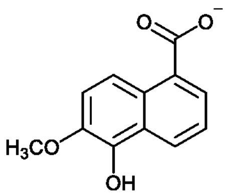 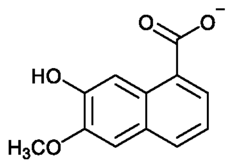 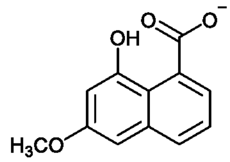

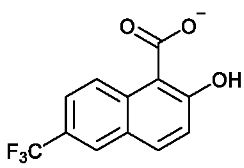 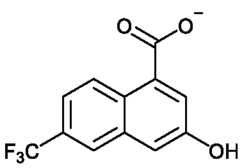 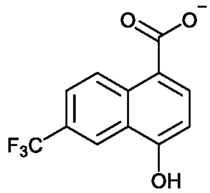 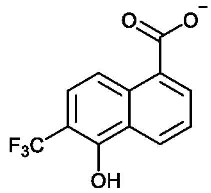

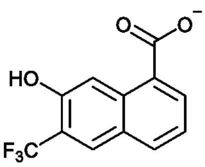 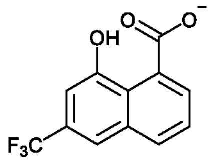

For example, the compound represented by Chemical Formula 1 may be represented by any one of the following Chemical Formulae 1-1 to 1-4:

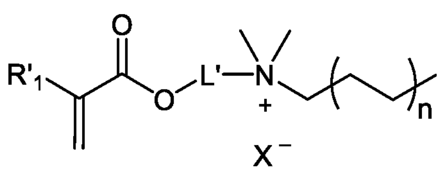

**1-1**

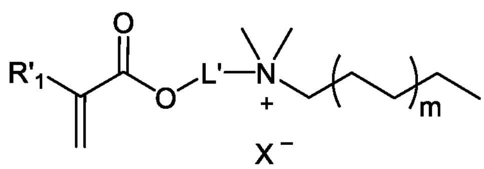

**1-2**

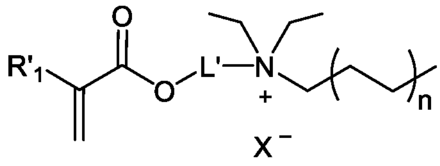

1-3

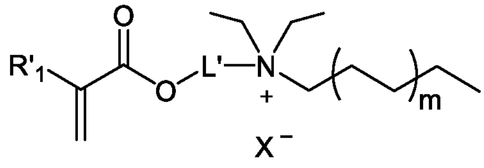

1-4

in Chemical Formulae 1-1 to 1-4,

R'$_1$ may be hydrogen or methyl,
L' may be methylene, or ethylene,
$X^-$ is the same as defined in Chemical Formula 1,
n is an integer of 2 to 7, and
m is an integer of 2 to 6.

In other words, in Chemical Formulae 1-1 to 1-4,

n may be 2, 3, 4, 5, 6, or 7, and
m may be 2, 3, 4, 5, or 6.

Meanwhile, the compound is any one selected from the group consisting of the following compounds:

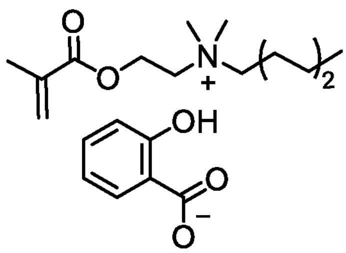

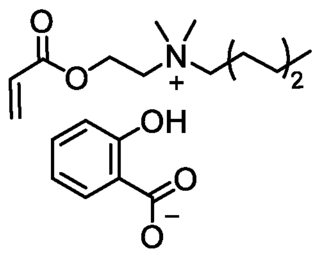

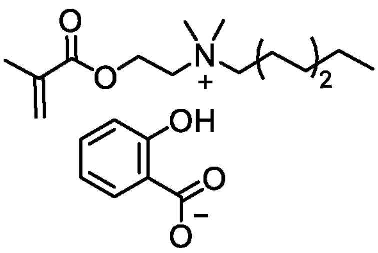

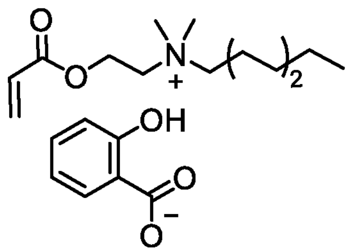

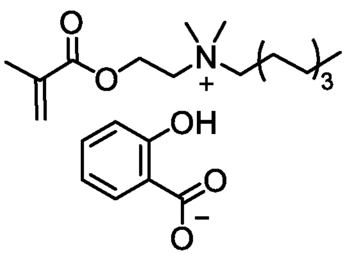

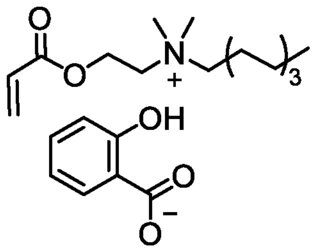

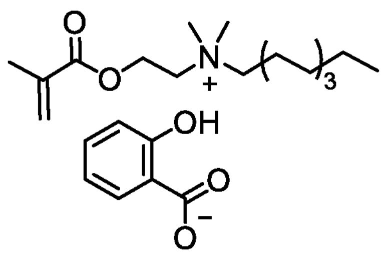

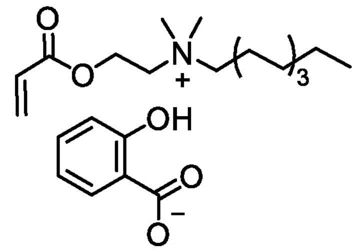

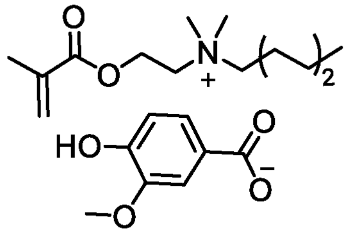

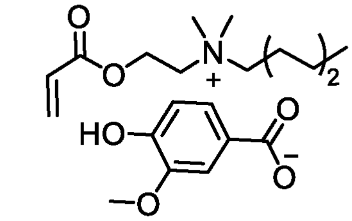

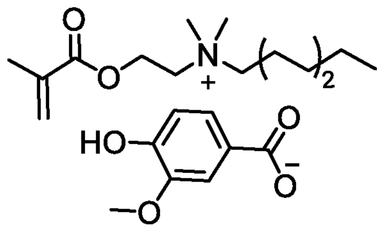

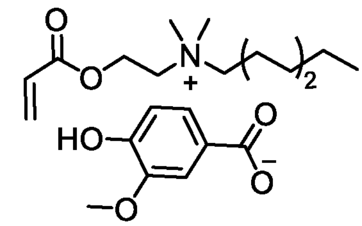

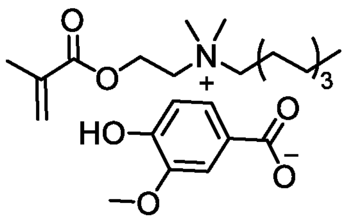

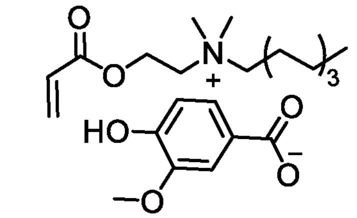

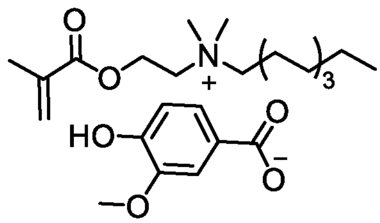

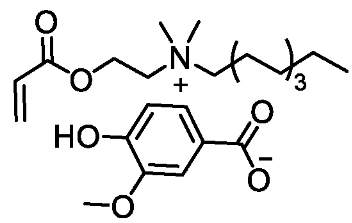

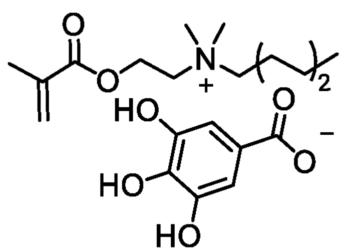

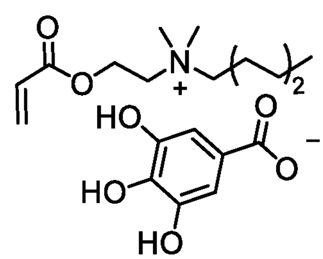

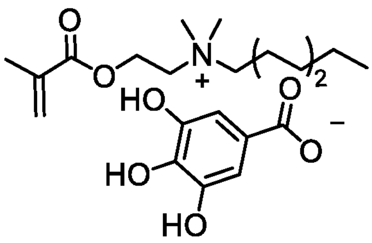

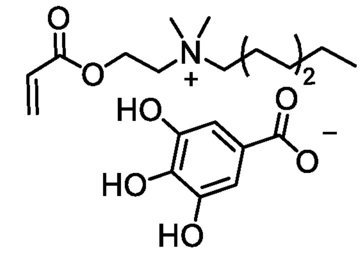

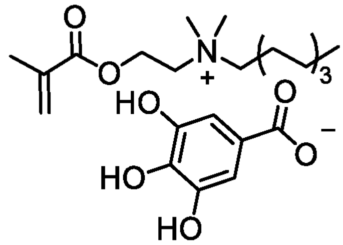

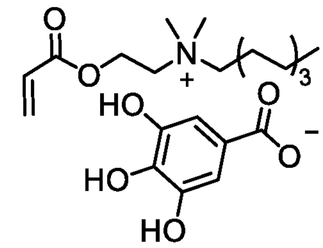

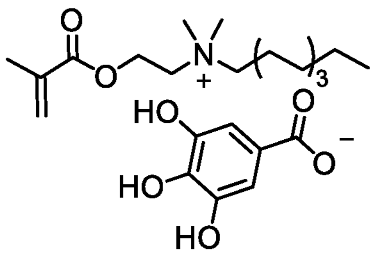

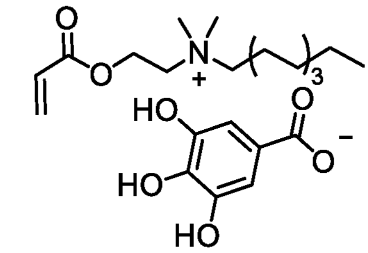

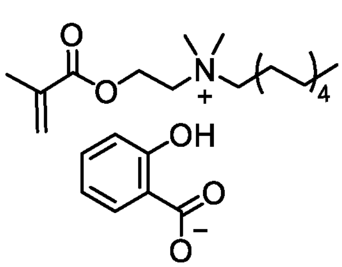

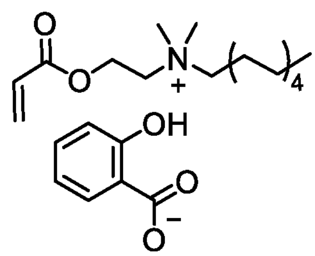

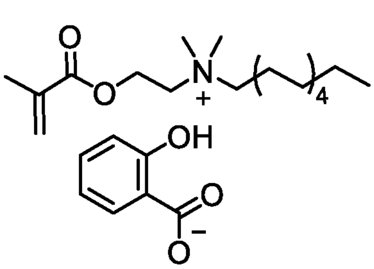

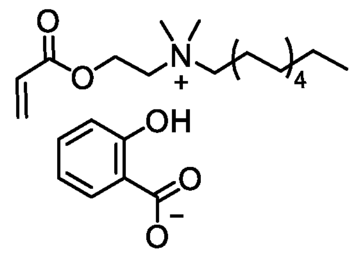

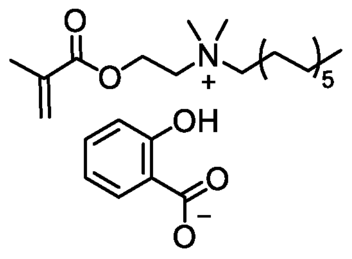

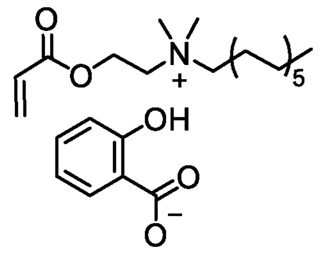

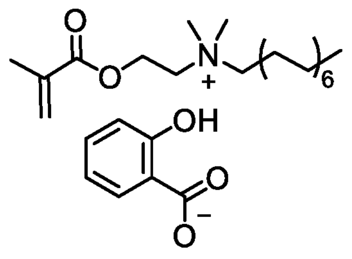

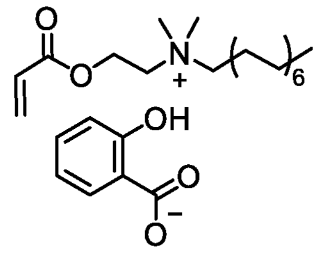

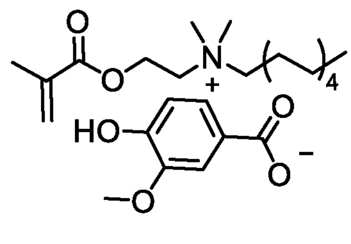

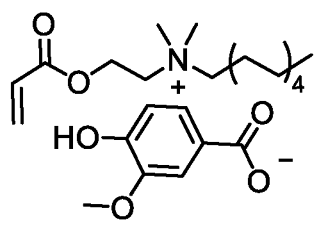

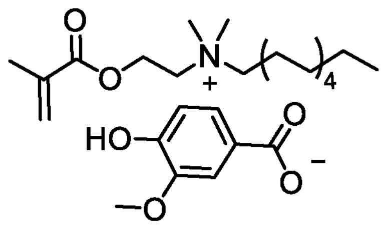

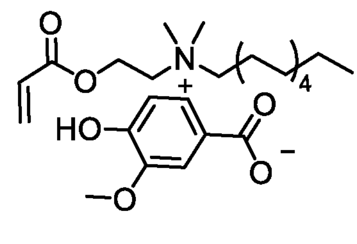

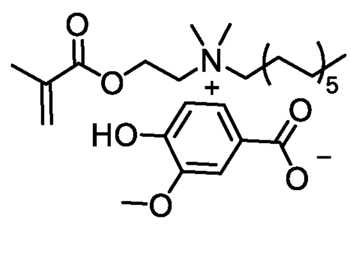

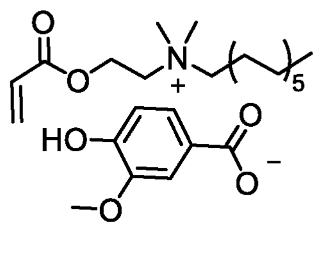

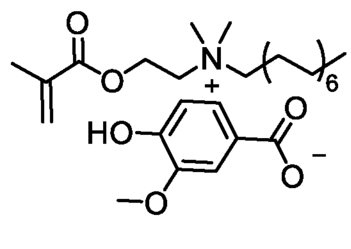

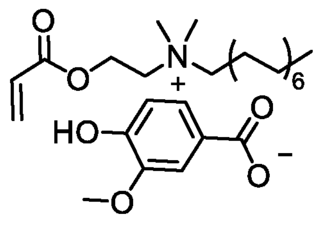

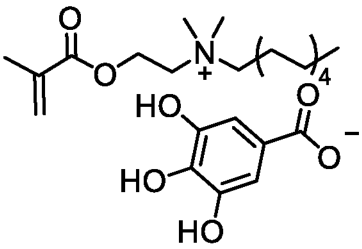

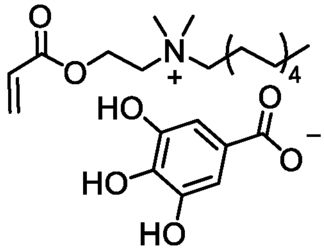

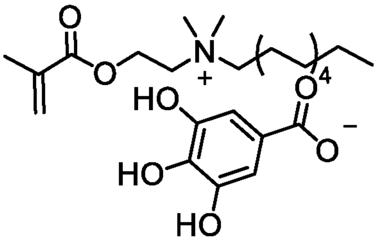

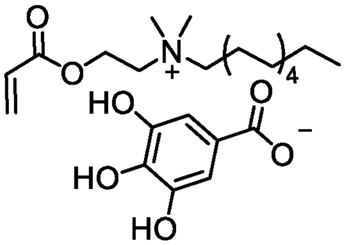

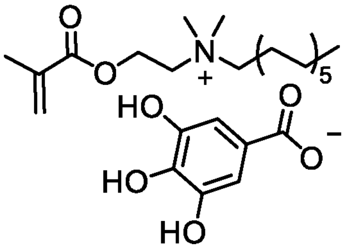

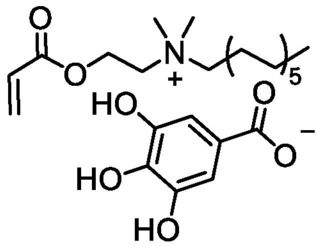

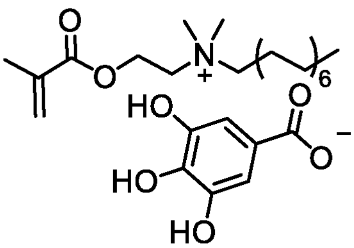

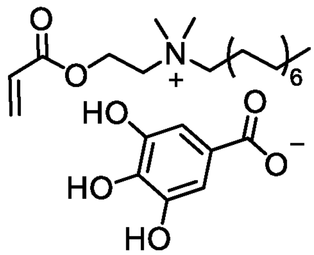

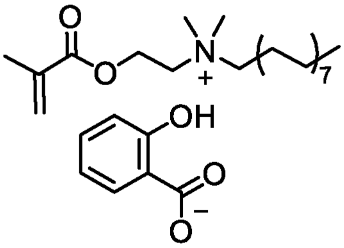

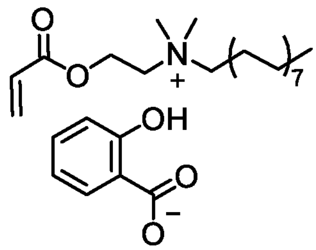

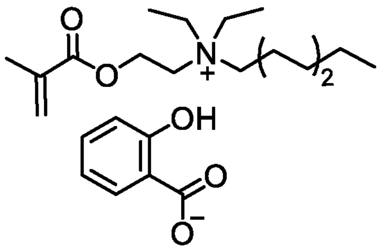

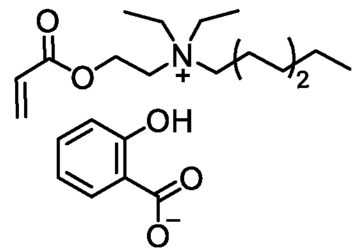

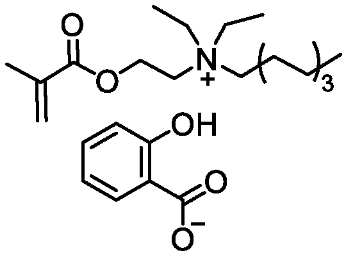

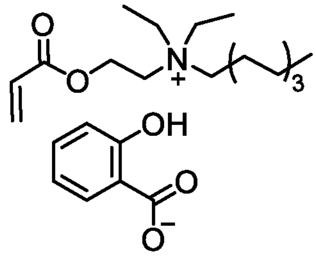

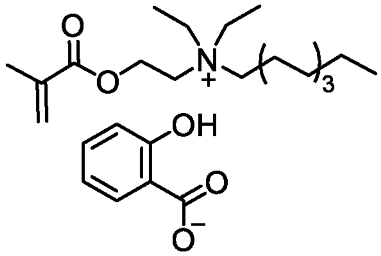

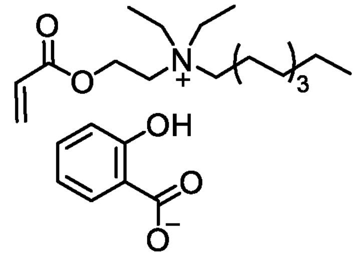

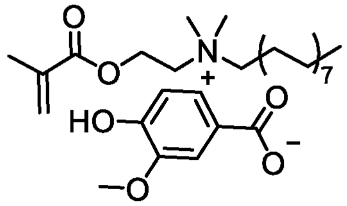

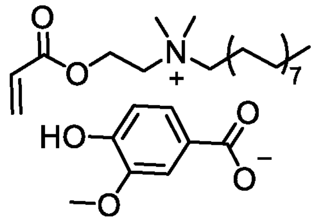

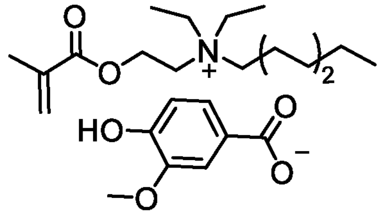

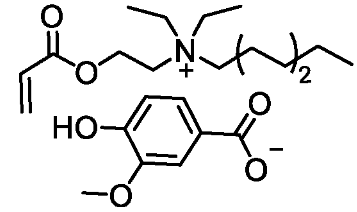

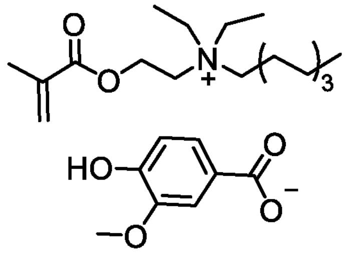

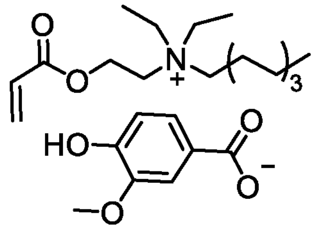

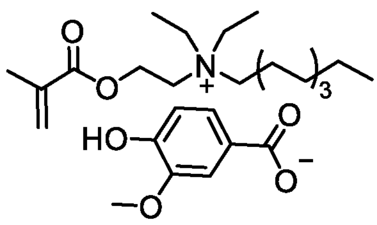

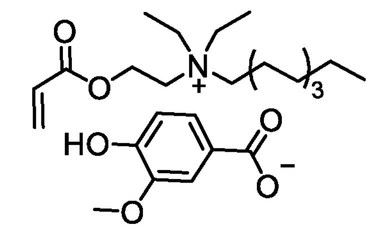

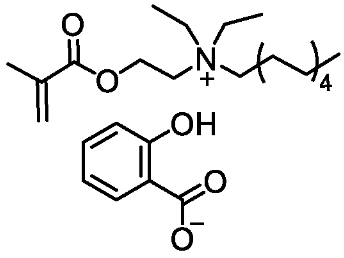

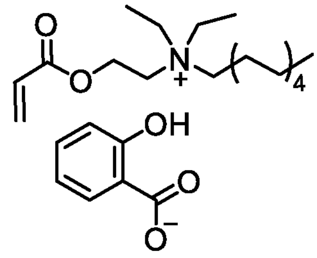

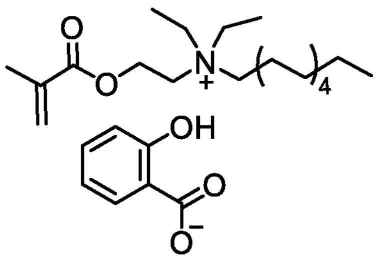

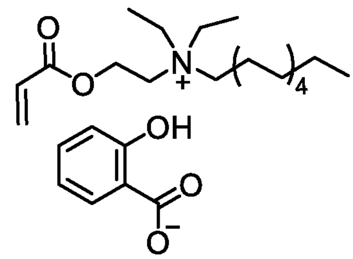

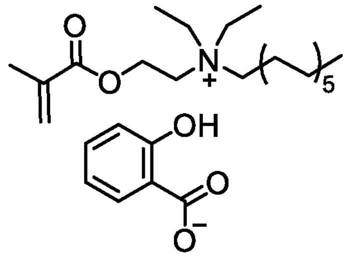

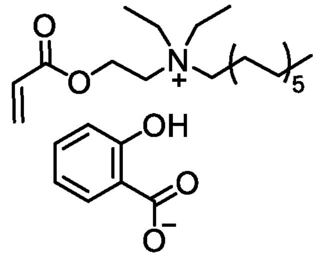

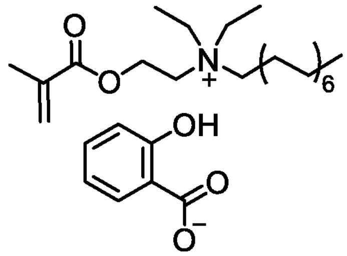

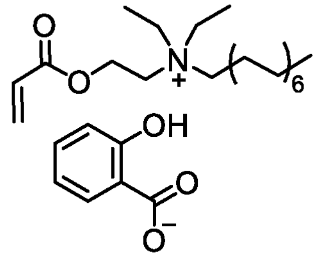

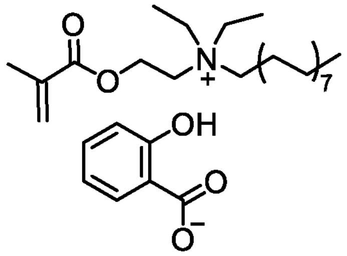

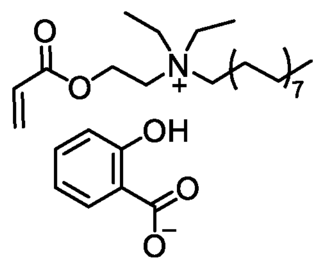

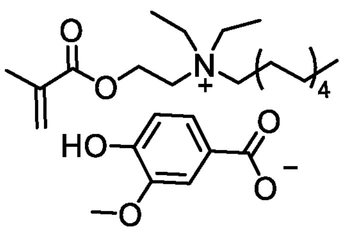

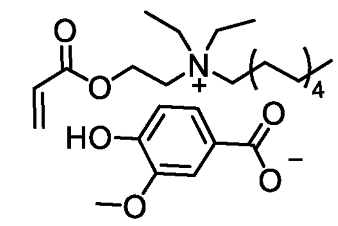

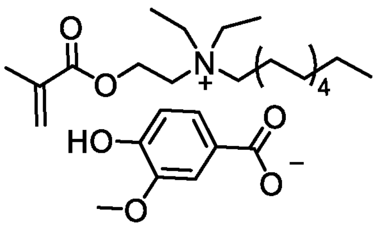

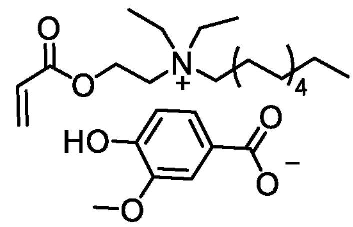

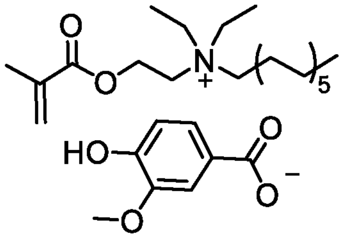

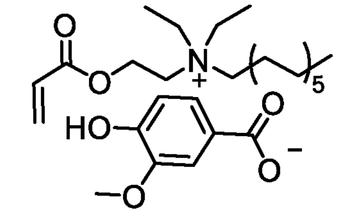

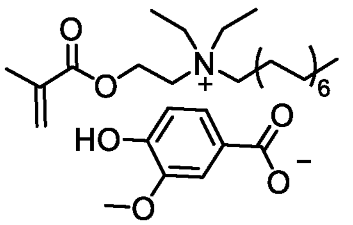

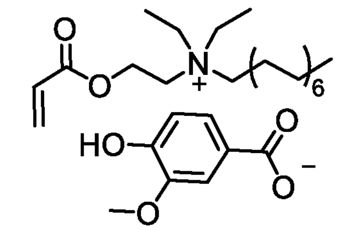

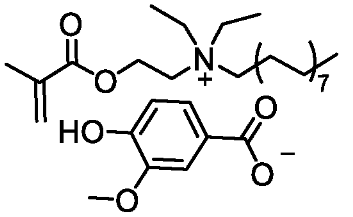

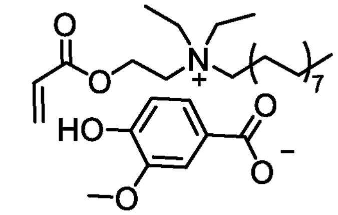

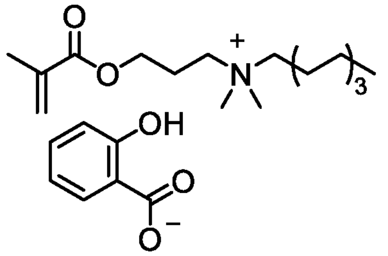

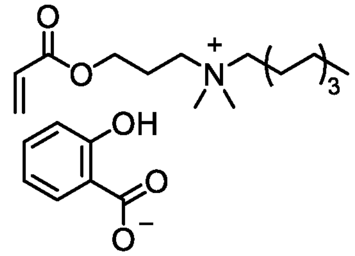

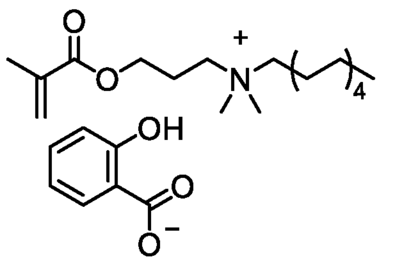

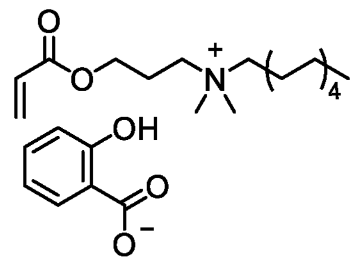

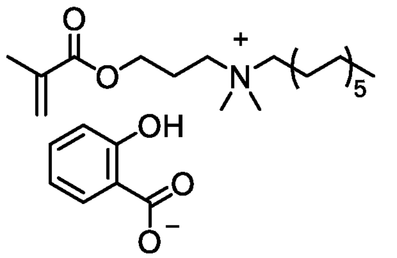

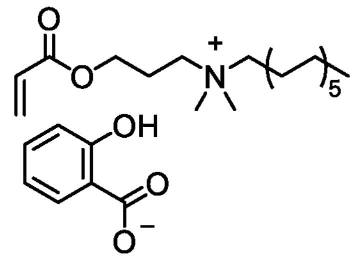

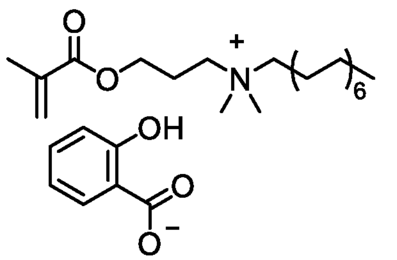

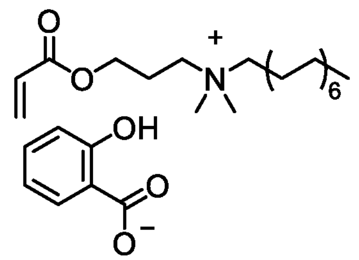

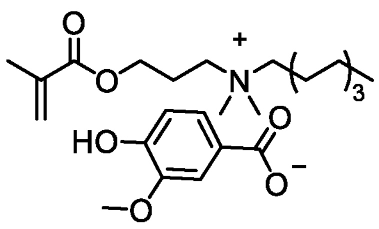

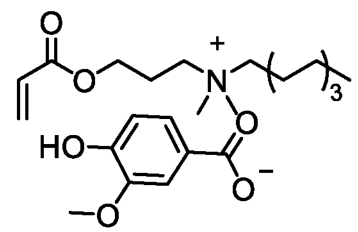

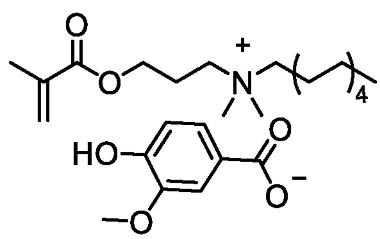

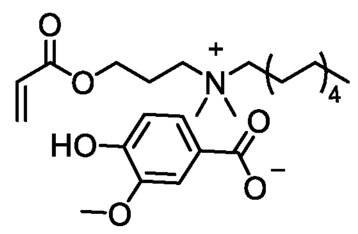

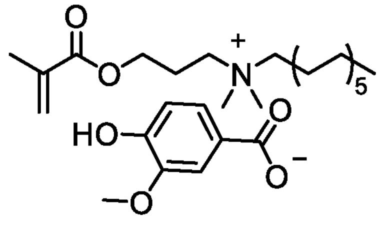

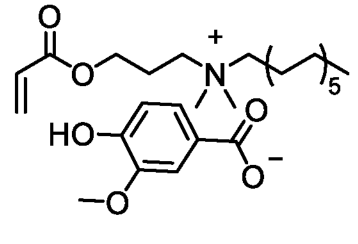

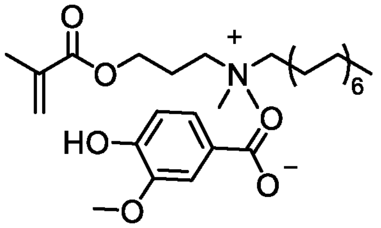

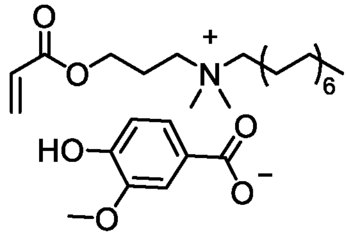
.

Meanwhile, the compound represented by Chemical Formula 1 may be prepared by a preparation method as in the following Reaction Scheme 1:

[Reaction Scheme 1]

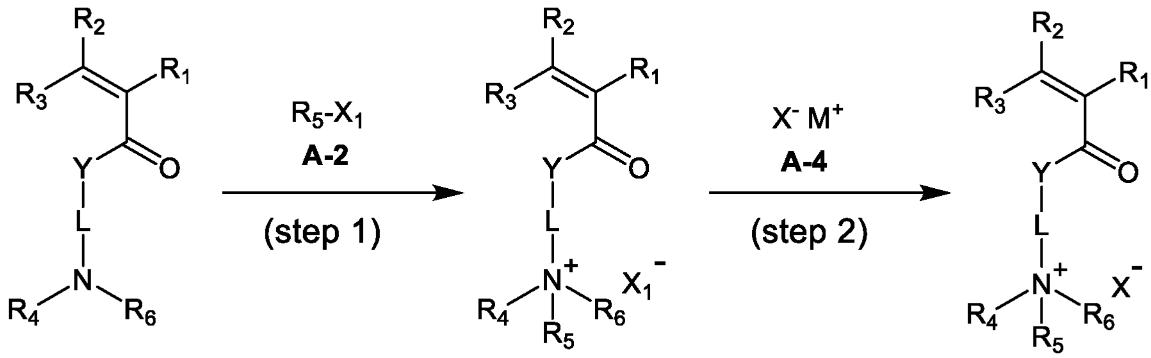


**A-1** **A-3** **1**

in Reaction Scheme 1, $X_1$ is halogen, more preferably, bromo or chloro, M is an alkali metal, and descriptions of the remaining substituents are the same as defined in Chemical Formula 1.

Step 1 in Reaction Scheme 1 is a step of preparing a quaternary ammonium salt compound A-3 by reacting a tertiary ammonia compound A-1 with a halide compound A-2, and step 2 is an anion substitution reaction step for substituting the halogen anion of the compound A-3 prepared in step 1 with the desired $X^-$ ion. The above preparation method may be more specified in Preparation Examples to be described later.

Further, the compound may exhibit excellent antibacterial effects against microorganisms, particularly, at least one of Gram-positive bacteria and Gram-negative bacteria.

More specifically, the compound may exhibit the antibacterial property against one or more kinds of bacteria, categorized as the Gram-positive bacteria. Alternatively, the compound may exhibit the antibacterial property against one or more kinds of bacteria, categorized as the Gram-negative bacteria. Alternatively, the compound may exhibit the antibacterial property against one or more kinds of bacteria, categorized as the Gram-negative bacteria and one or more kinds of bacteria, categorized as the Gram-positive bacteria.

In this regard, the Gram-negative bacteria against which the compound may exhibit the antibacterial property may be *Proteus mirabilis,* or *Escherichia coli,* and the Gram-positive bacteria against which the compound may exhibit the antibacterial property may be *Enterococcus faecalis,* but are not limited thereto. More preferably, the compound may

exhibit the antibacterial property against both of the Gram-positive bacteria and the Gram-negative bacteria. In this regard, the meaning that the compound exhibits the antibacterial property may be confirmed by an antibacterial rate of 50% or more, as measured in an antibacterial property test using absorbance, described later.

Here, *Proteus mirabilis* is a Gram-negative, rod-shaped, facultatively anaerobic or aerobic bacterium, and distributed in various environments, and may infect the respiratory tract or skin of humans and animals to cause urinary tract-related diseases. In particular, it is known that when a person is infected with the *Proteus mirabilis,* it may cause urinary tract infection or acute pyelonephritis. In addition, *Proteus mirabilis* alkalizes urine to allow ammonia to be excreted, which may cause odor.

Specifically, the antibacterial property of the compound against *E.coli* may be evaluated by measuring absorbance, and an antibacterial rate of the compound against *E.coli,* as calculated by the following Equation 1, may be 70% or more.

[Equation 1]

$$\text{Antibacterial rate (\%)} = (1 - A_s/A_0) \times 100$$

in the equation,

$A_s(A_{sample})$ represents absorbance of the culture medium containing the bacteriostatic substance at a wavelength of 600 nm, and

$A_0(A_{reference})$ represents absorbance of the *E.coli* pure culture medium without the bacteriostatic substance at a wavelength of 600 nm.

More preferably, the antibacterial rate of the compound against *E.coli,* as calculated by Equation 1, may be 70% or more, 70.6% or more, 75.5% or more, 80% or more, 90% or more, 95% or more, 95.3% or more, 95.8% or more, 96% or more, 97% or more, 97.3% or more, 98% or more, 98.1% or more, and 100% or less.

Evaluation of the antibacterial property of the compound against *Proteus mirabilis* and *Enterococcus faecalis* may also be performed in the same manner as in the evaluation of the antibacterial property of the compound against *E.coli.*

**Antibacterial agent**

Meanwhile, according to another aspect, provided is an antibacterial agent including the above-described compound represented by Chemical Formula 1. The antibacterial agent may include the compound having the excellent bacterial growth-inhibitory effect as described above, thereby exhibiting excellent antibacterial property.

In this regard, the antibacterial agent may be mixed in an article requiring antibacterial property, or applied or coated onto the article. It may be applied to various household chemical products, in which harmful bacteria grow easily, without limitation, for examples, articles requiring such antibacterial property, such as humidifiers, water tanks, refrigerators, air washers, aquariums, air purifiers, agricultural films, freshness-keeping materials, containers for processed foods, and packaging materials for electronic parts, etc.

Further, the antibacterial agent including the compound may be prepared in various forms, such as an antibacterial coating composition, an antibacterial resin, an antibacterial plastic, etc., depending on the application required. Furthermore, the antibacterial agent may further include other types of resins and/or solvents to facilitate its mixing with or application onto the antibacterial articles.

Hereinafter, the actions and effects of the present invention will be described in more detail with reference to the specific exemplary embodiments of the present invention. However, these exemplary embodiments are provided only for illustrating the present invention, and the scope of the present invention is not defined thereby.

**Preparation Example A: Preparation of Compound A**

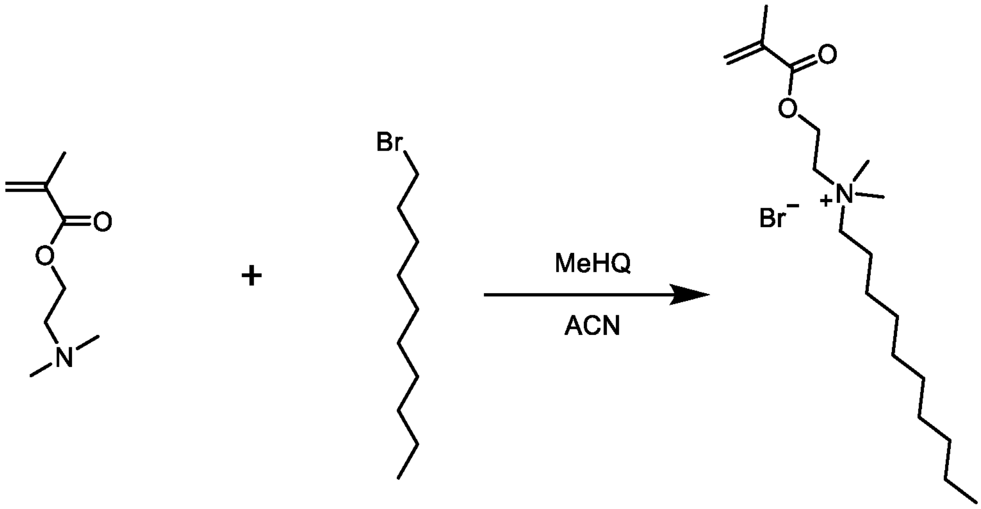


**A**

7.86 g of 2-(dimethylamino)ethyl methacrylate and 11.06 g of bromodecane were put in 30 mL of acetonitrile (ACN), followed by stirring. Thereafter, 4 mg of p-methoxyphenol (4-methoxyphenol; MeHQ) was added thereto, and a reflux reaction was allowed at 60 °C for 24 hours. After the reaction was completed, the reaction product was added to 300 mL of diethyl ether, and precipitated by stirring, and filtered to obtain Compound A. As a result of MALDI-TOF mass spectrometry and $^1$H NMR analysis of the obtained compound A, a value corresponding to the cation of Compound A was detected. Meanwhile, MS spectrum and $^1$H NMR spectrum of Compound A are shown in FIGS. 1 and 2, respectively.

$[MS\text{-}H]^+$ = 298

$^1$H NMR (500MHz, DMSO-$d_6$,δ[ppm]): 6.08(1H), 5.77(1H), 4.52(2H), 3.69(2H), 3.35(2H), 3.09(6H), 1.91(3H), 1.67(2H), 1.25(14H), 0.96(3H)

**Preparation Example B: Preparation of Compound B**

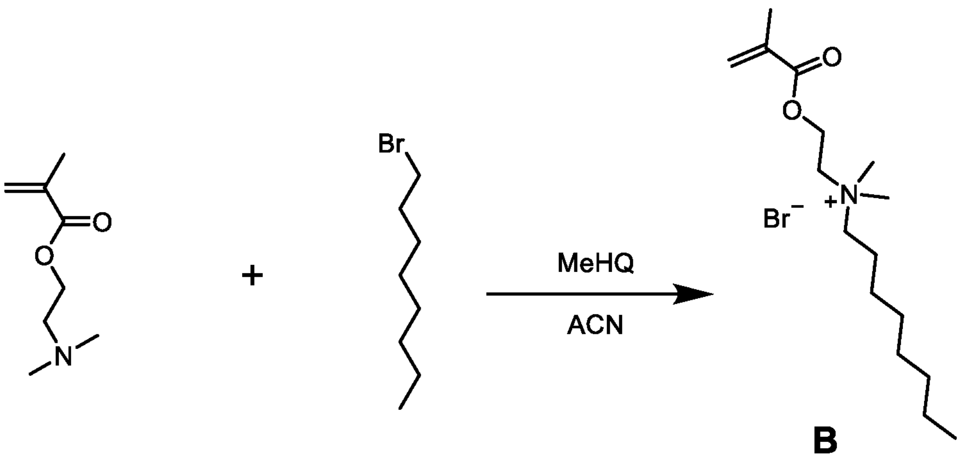


**B**

Compound B was synthesized in the same manner as in Preparation Example A, except that bromooctane was used instead of bromodecane in Preparation Example A.
$[MS\text{-}H]^+$ = 270

**Preparation Example C: Preparation of Compound C**

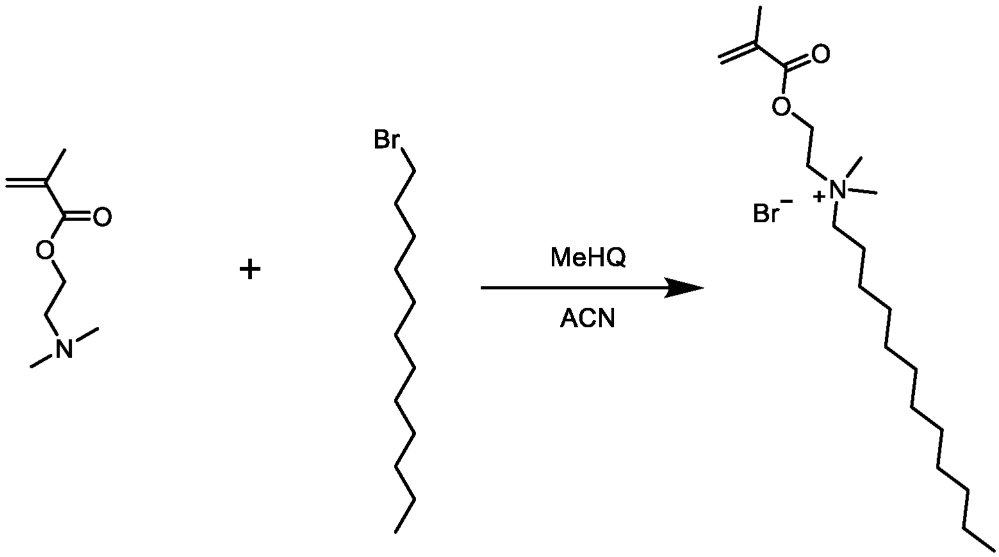


C

Compound C was synthesized in the same manner as in Preparation Example A, except that bromododecane was used instead of bromodecane in Preparation Example A.
$[MS-H]^+$ = 326

**Preparation Example D: Preparation of Compound D**

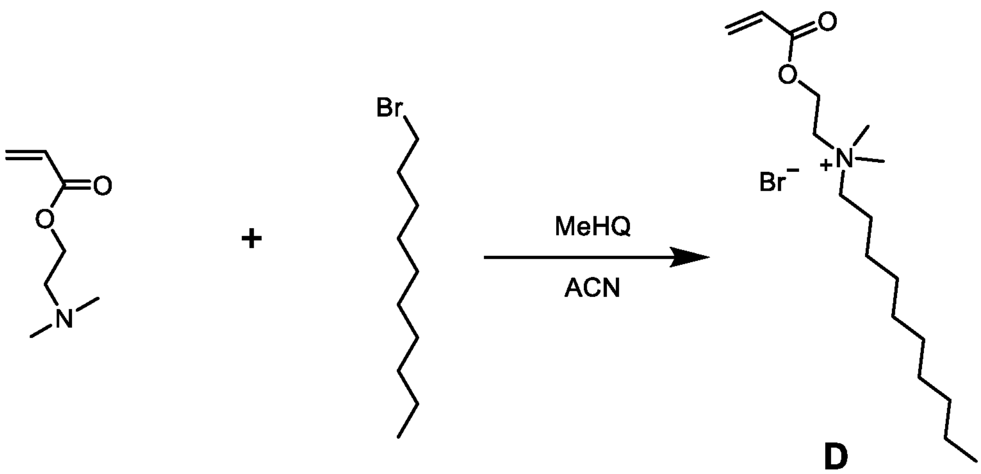


D

7.16 g of 2-(dimethylamino)ethyl acrylate and 11.06 g of bromodecane were put in 30 mL of acetonitrile (ACN), followed by stirring. Thereafter, 4 mg of p-methoxyphenol (MeHQ) was added thereto, and a reflux reaction was allowed at 60 °C for 24 hours. After the reaction was completed, the reaction product was added to 250 mL of diethyl ether, and precipitated by stirring, and filtered to obtain Compound A. As a result of MALDI-TOF mass spectrometry and $^1H$ NMR analysis of the obtained compound D, a value corresponding to the cation of Compound D was detected.

$[MS-H]^+$ = 284

$^1H$ NMR (500MHz, DMSO-$d_6$,$\delta$[ppm]): 6.48(1H), 6.11(1H), 5.94(1H), 4.67(2H), 4.13(2H), 3.58(2H), 3.48(6H), 1.74(2H), 1.27(14H), 0.86(3H)

**Preparation Example E: Preparation of Compound E**

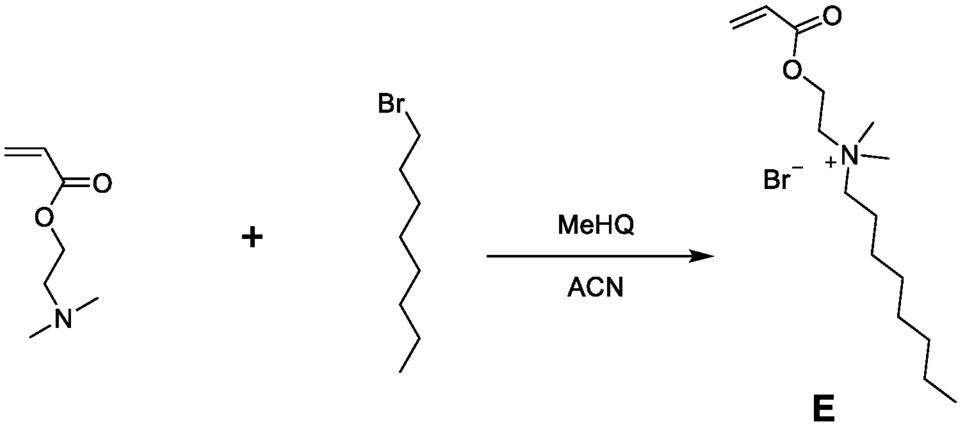


**E**

Compound E was synthesized in the same manner as in Preparation Example D, except that bromooctane was used instead of bromodecane in Preparation Example D.
$[MS-H]^+ = 256$

**Preparation Example 1: Preparation of Compound 1**

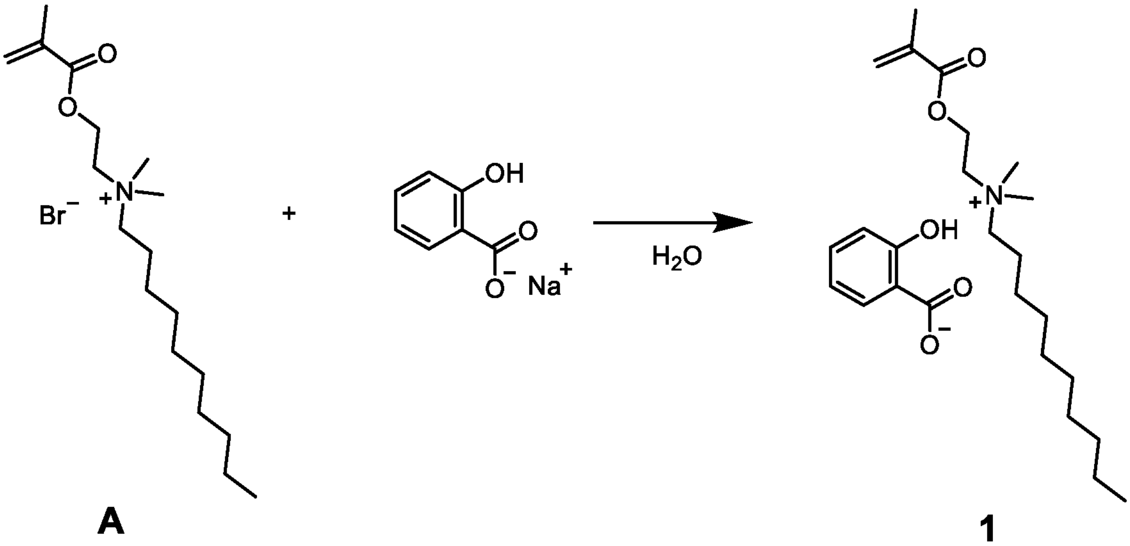


**A** **1**

20 g of Compound A synthesized in Preparation Example A was dissolved in 100 mL of DI water, and 11.3 g of sodium salicylate dissolved in 50 mL of water was mixed and stirred at room temperature for 24 hours to perform an anion substitution reaction. Then, an organic layer was extracted from the reaction product with ethyl acetate (EA)/DI water to remove the solvent, and recrystallization was performed with EA to obtain Compound 1. As a result of $^1H$ NMR analysis, a peak corresponding to the anion of Compound 1 was observed, and as a result of inorganic analysis, Compound 1 showed a remarkably low Br content, as compared to Compound A, indicating that the substitution normally occurred. Meanwhile, $^1H$ NMR spectrum and mass spectrum of Compound 1 are shown in FIGS. 3 and 4, respectively, and the results of measuring the Br contents of Compound 1 and Compound A using a combustion ion chromatography (C-IC) analyzer are shown in Table 1 below.

$[MS-H]^+ = 298$
$[M-H]^- = 137$

$^1H$ NMR (500MHz, DMSO-$d_6$,δ[ppm]): 7.63(1H), 7.09(1H), 6.58(2H), 6.08(1H), 5.76(1H), 4.53(2H), 3.69(2H), 3.32(2H), 3.08(6H), 1.91(3H), 1.67(2H), 1.25(14H), 0.86(3H)

[Table 1]

| | Br content (wt%) |
|---|---|
| Compound A | 15.4 |
| Compound 1 | 2.2 |

**Preparation Example 2: Preparation of Compound 2**

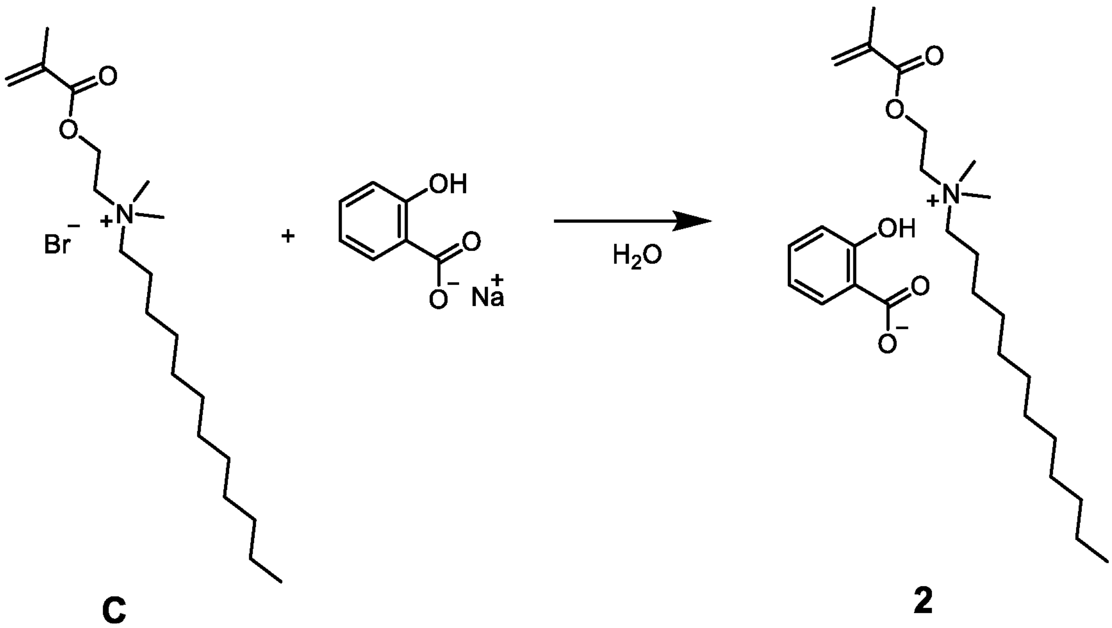


C

2

Compound 2 was synthesized in the same manner as in Preparation Example 1, except that Compound C prepared in Preparation Example C was used instead of Compound A in Preparation Example 1.

[MS-H]$^+$ = 326

[M-H]- = 137

**Preparation Example 3: Preparation of Compound** 3

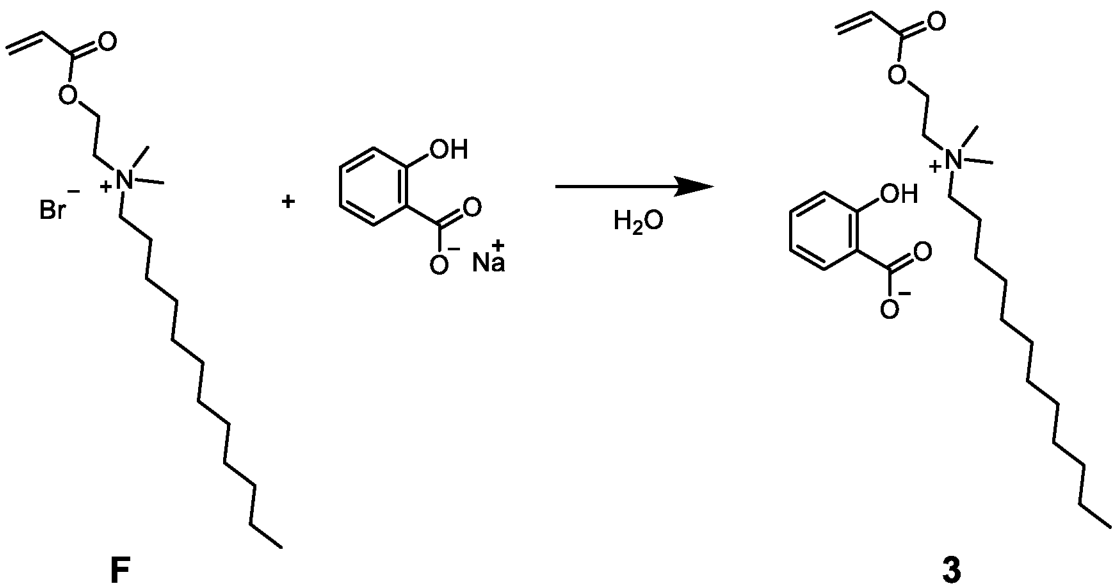


F 3

Compound 3 was synthesized in the same manner as in Preparation Example 1, except that compound F (N-(2-(acryloyloxy)ethyl)-N,N-dimethyldodecane-1-aminium bromide) was used instead of Compound A in Preparation Example 1.

[MS-H]$^+$ = 312

[M-H]- = 137

**Preparation Example 4: Preparation of Compound 4**

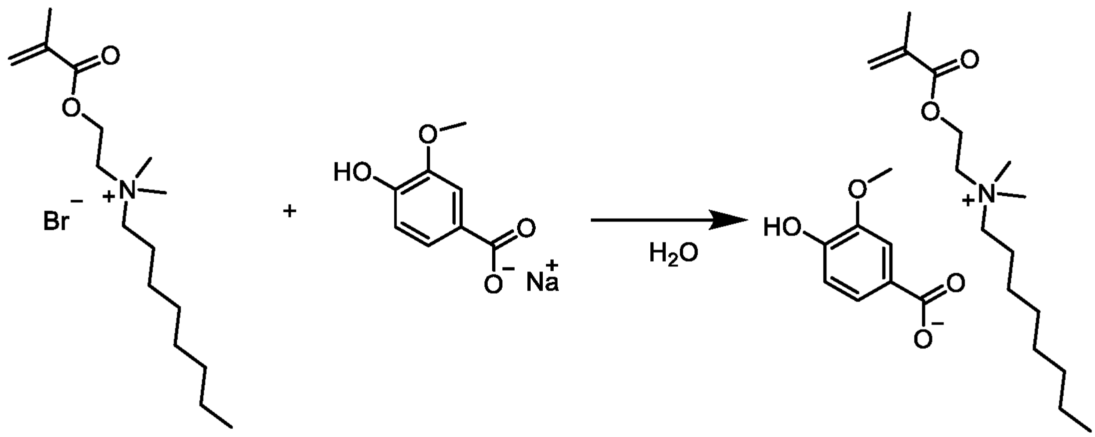


B 4

Compound 4 was synthesized in the same manner as in Preparation Example 1, except that compound B prepared in Preparation Example B was used instead of Compound A and sodium vanillate was used instead of sodium salicylate in Preparation Example 1.

[MS-H]$^+$ = 284

[M-H]- = 167

**Preparation Example 5: Preparation of Compound 5**

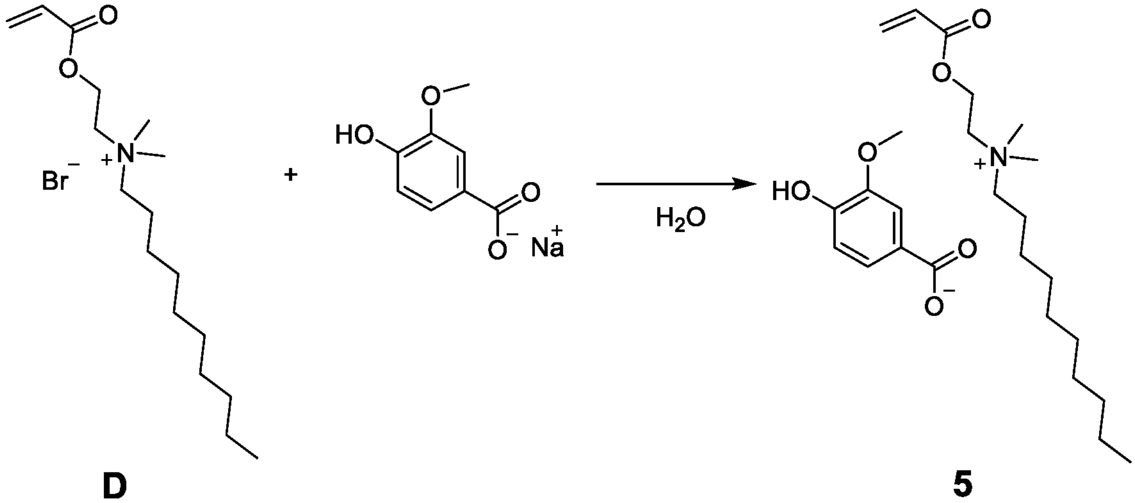


D 5

Compound 5 was synthesized in the same manner as in Preparation Example 1, except that Compound D prepared in Preparation Example D was used instead of Compound A and sodium vanillate was used instead of sodium salicylate in Preparation Example 1.

$[MS-H]^+ = 256$

[M-H]- = 167

**Preparation Example 6: Preparation of Compound 6**

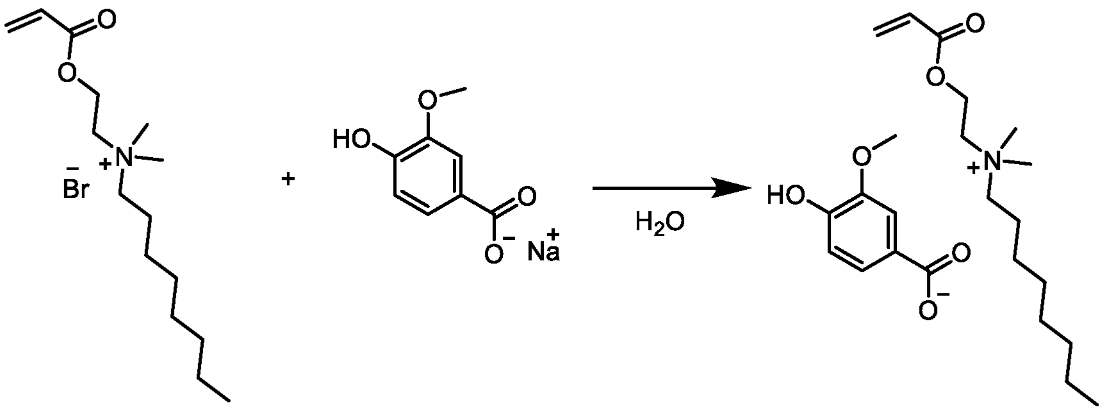


E 6

Compound 6 was synthesized in the same manner as in Preparation Example 1, except that Compound E prepared in Preparation Example E was used instead of Compound A and sodium vanillate was used instead of sodium salicylate in Preparation Example 1.

$[MS-H]^+ = 284$

[M-H]- = 167

**Experimental Example- Evaluation of antibacterial property**

Unless otherwise indicated, the following property evaluation was performed at constant temperature and constant humidity (23±1°C, relative humidity 50±10%).

(1) Evaluation of antibacterial property against *E.coli*

25 mL of a nutrient broth culture medium inoculated with *E. coli* (ATCC 25922) at 3000 CFU/mL was transferred to a 50 mL conical tube, and then the antibacterial compounds (bacteriostatic substances) prepared in Preparation Examples and Comparative Preparation Examples were injected in each amount described in Table 2 below, followed by sufficiently mixing. Thereafter, incubation was performed for 16 hours in a shaking incubator (Visiontech, VS-37SIF) maintained at 35 °C.

The incubated solution was diluted to 1/5 using 1 x PBS buffer solution, and absorbance at the wavelength of 600 nm was measured for the diluted solution using a UV-Vis Spectrophotometer (K Lab, Optizen POP). In addition, *E. coli* (ATCC 25922) was cultured in a pure culture medium without the antibacterial compound for 16 hours in a shaking incubator (VISION TECH, VS-37SIF) maintained at 35 °C, which was prepared as a control, and absorbance at the wavelength of 600 nm was measured in the same manner as above.

Then, antibacterial rates (%) of *E.coli* (ATCC 25922) were calculated according to the following Equation 1, and the results are shown in Table 2 below.

[Equation 1]

$$\text{Antibacterial rate (\%)} = (1 - A_s/A_0) \times 100$$

in the equation,

$A_s$($A_{sample}$) represents absorbance of the culture medium containing the bacteriostatic substance at a wavelength of 600 nm, and

$A_0$($A_{reference}$) represents absorbance of the *E.coli* pure culture medium without the bacteriostatic substance at a wavelength of 600 nm.

[Table 2]

| | Kind of compound | Injection amount of compound (g) | Antibacterial rate (%) |
|---|---|---|---|
| Example 1 | Compound 1 | 0.01 | 95.3 |
| Example 2 | Compound 2 | 0.01 | 98.1 |
| Example 3 | Compound 3 | 0.01 | 97.3 |
| Example 4 | Compound 4 | 0.01 | 75.5 |
| Example 5 | Compound 5 | 0.01 | 95.8 |
| Example 6 | Compound 6 | 0.01 | 70.6 |
| Comparative Example 1 | Compound B | 0.01 | 69.2 |
| Comparative Example 2 | Compound E | 0.01 | 62.3 |

Referring to Table 2, it can be seen that the compounds of Examples, each having the conjugate base of aromatic acid, exhibited the improved antibacterial property against *E. coli,* as compared to the compounds of Comparative Examples, each having the halogen anion. In particular, when comparing (Compound 4 of Example 4 and Compound B of Comparative Example 1) and (Compound 6 of Example 6 and Compound E of Comparative Example 2), respectively, the antibacterial rates of the compounds of Examples, each having the conjugate base of aromatic acid as an anion, were found to be significantly improved, even though they have the same cation structure.

Accordingly, it can be seen that the compound represented by Chemical Formula 1, in which the quaternary ammonium cation and the anion of the conjugate base of aromatic acid are bound, exhibits the excellent antibacterial property against at least one of Gram-positive bacteria and Gram-negative bacteria.

## Claims

1. A compound represented by the following Chemical Formula 1:

[Chemical Formula 1]

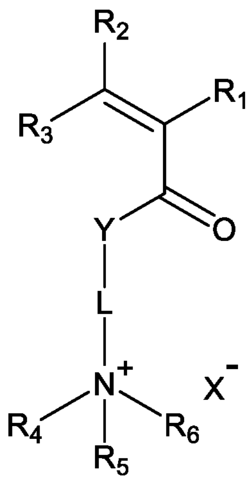

in Chemical Formula 1,

Y is O, S, or $N(R_7)$,
L is a single bond, an alkylene having 1 to 10 carbon atoms, or an arylene having 6 to 60 carbon atoms,
$R_1$ to $R_3$ are each independently hydrogen or methyl,
$R_4$ to $R_7$ are each independently hydrogen, a substituted or unsubstituted alkyl having 1 to 20 carbon atoms, or a substituted or unsubstituted aryl having 6 to 60 carbon atoms, and
$X^-$ is a conjugate base of an aromatic acid having 6 to 20 carbon atoms, which is substituted with one or more hydroxyl groups,
wherein the conjugate base of the aromatic acid is optionally further substituted with one or more substituents selected from the group consisting of a halogen, an alkyl having 1 to 4 carbon atoms, a haloalkyl having 1 to 4 carbon atoms, and an alkoxy having 1 to 4 carbon atoms.

2. The compound of claim 1,
wherein Y is O.

3. The compound of claim 1,
wherein L is methylene, ethylene, or propylene.

4. The compound of claim 1,
wherein $R_1$ is hydrogen or methyl, and $R_2$ and $R_3$ are hydrogen.

5. The compound of claim 1,
wherein one of $R_4$ to $R_6$ is an alkyl having 5 to 20 carbon atoms, and the others are each independently an alkyl having 1 to 4 carbon atoms.

6. The compound of claim 5,
wherein one of $R_4$ to $R_6$ is an alkyl having 6 to 16 carbon atoms, and the others are each independently methyl or ethyl.

7. The compound of claim 1,
wherein $X^-$ is represented by the following Chemical Formula 2:

[Chemical Formula 2]

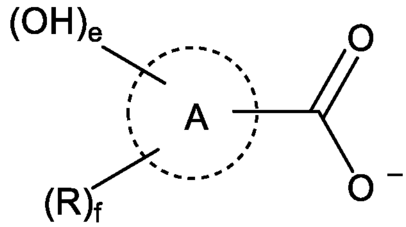

in Chemical Formula 2,

A is a benzene ring, or a naphthalene ring,
R is fluoro, bromo, chloro, iodo, methyl, ethyl, propyl, isopropyl, butyl, isobutyl, sec-butyl, tert-butyl, trifluoromethyl, methoxy, or ethoxy,
e is 1, 2, or 3, and
f is an integer of 0 to 5,
wherein when f is 2 or more, two or more R's are the same as or different from each other.

**8.** The compound of claim 1,
wherein $X^-$ is represented by any one of the following Chemical Formulae 2-1 to 2-6:

[Chemical Formula 2-1]

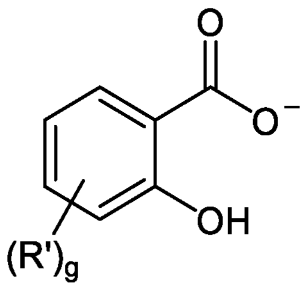

[Chemical Formula 2-2]

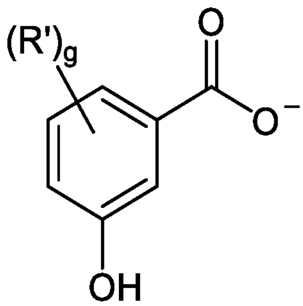

[Chemical Formula 2-3]

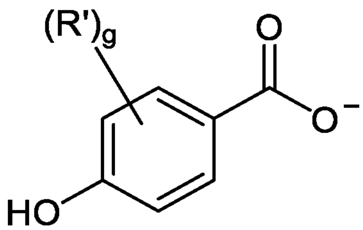

[Chemical Formula 2-4]

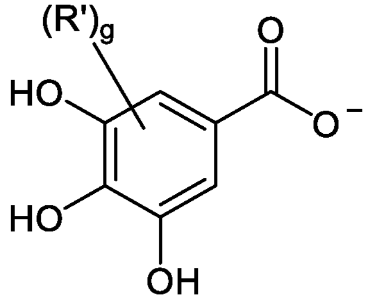

[Chemical Formula 2-5]

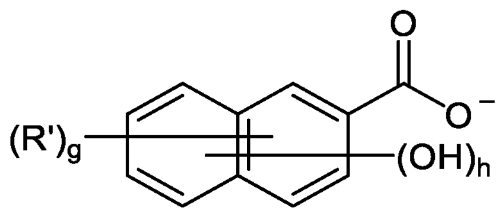

[Chemical Formula 2-6]

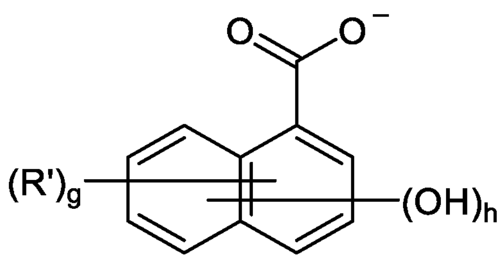

in Chemical Formulae 2-1 to 2-6,

R' is fluoro, bromo, chloro, methyl, trifluoromethyl, or methoxy,
g is 0, 1, or 2, and
wherein when g is 2, two R's are the same as or different from each other, h is 1 or 2.

**9.** The compound of claim 1,
wherein $X^-$ is any one selected from the group consisting of:

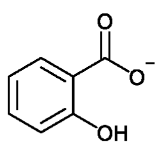
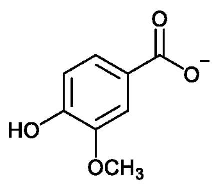
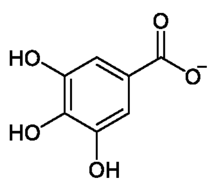
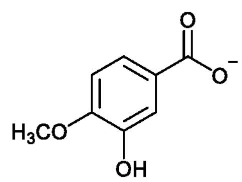
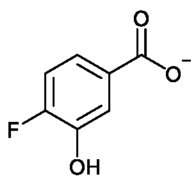
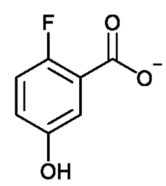
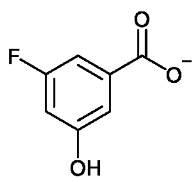
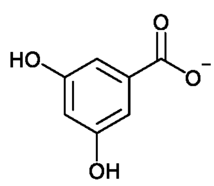

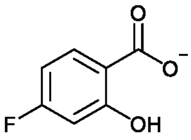 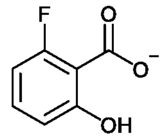 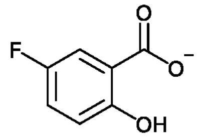 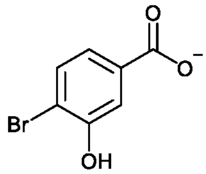

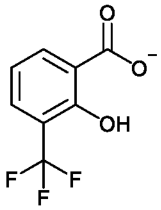 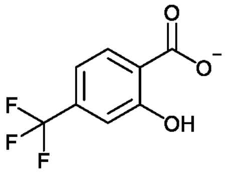 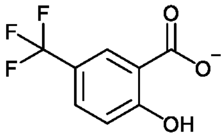 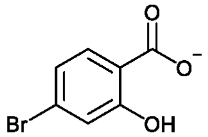

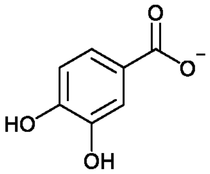 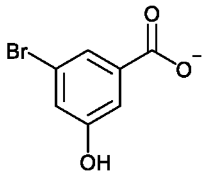 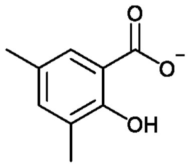 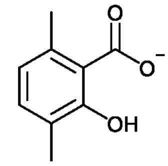

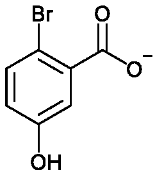 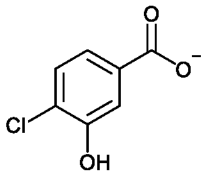 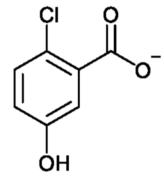 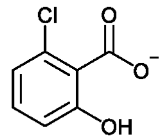

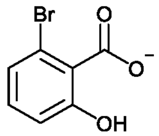 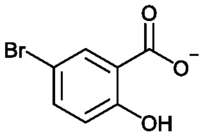 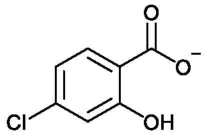 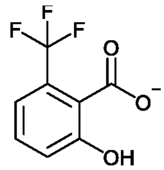

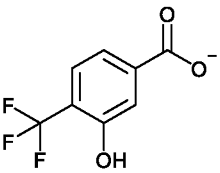 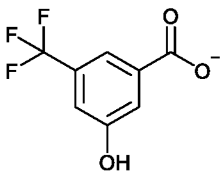 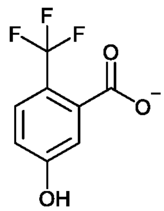 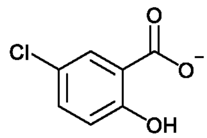

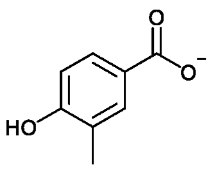 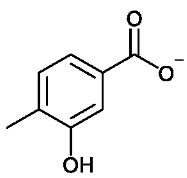 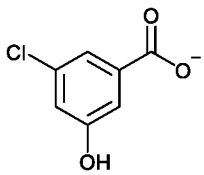

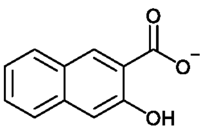 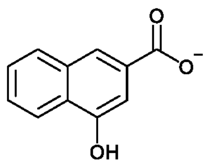 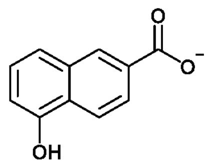 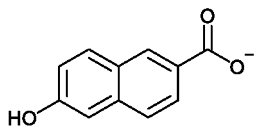

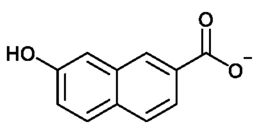 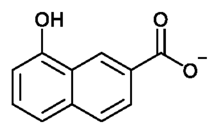 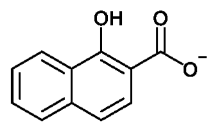 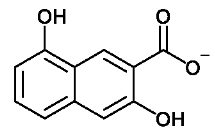

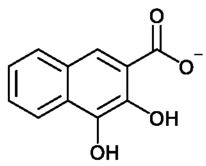 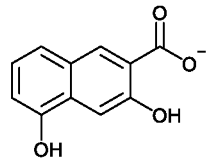 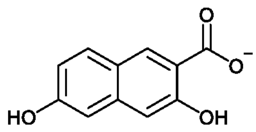 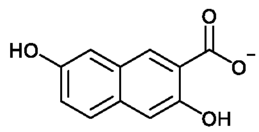

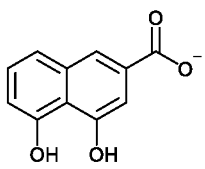 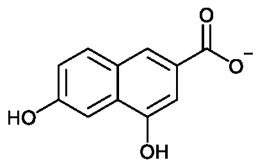 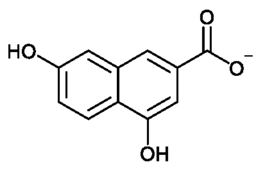 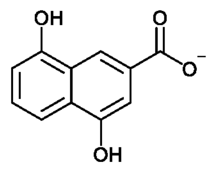

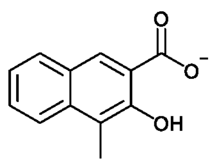 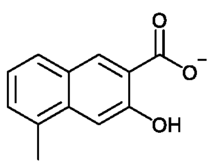 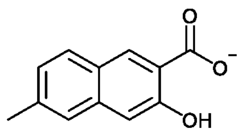 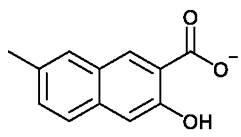

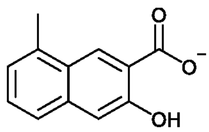 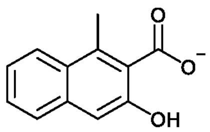 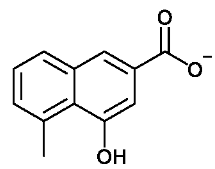 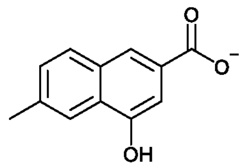

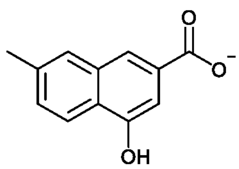 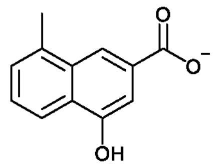 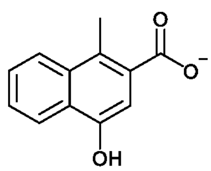 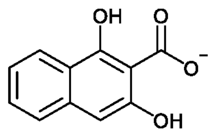

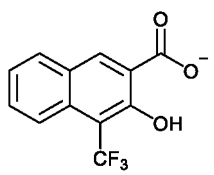 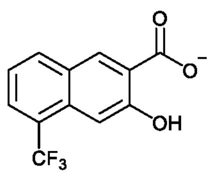 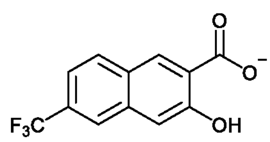 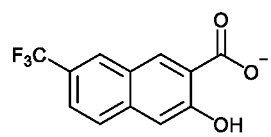

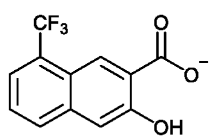 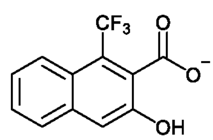 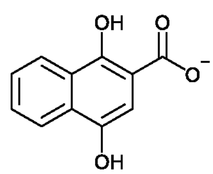

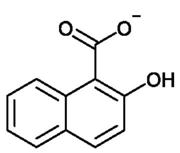 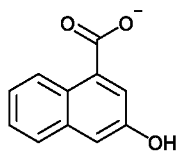 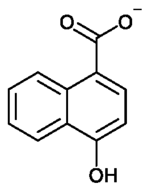 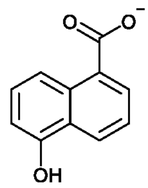

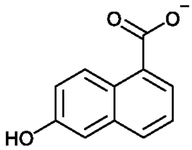 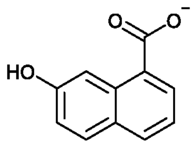 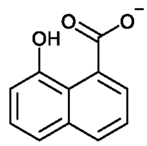

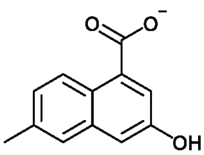 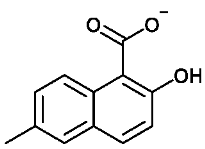 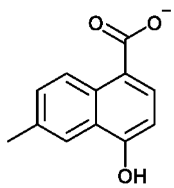 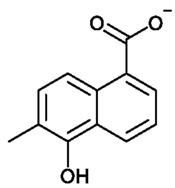

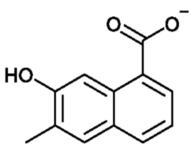 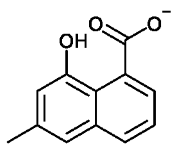 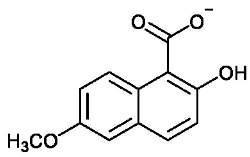 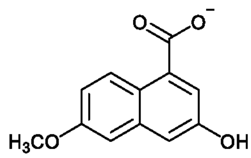

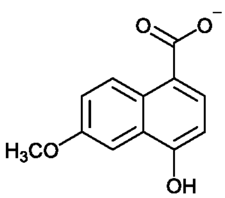 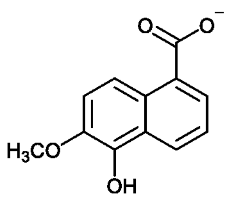 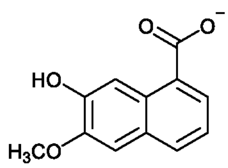 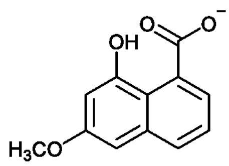

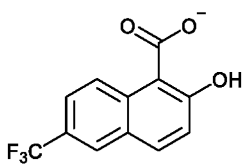 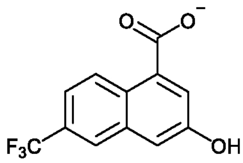 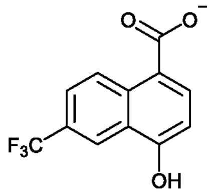 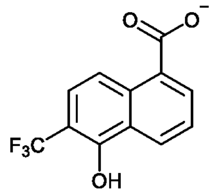

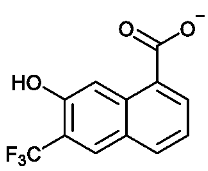 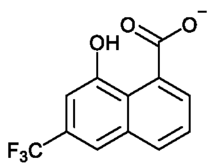

**10.** The compound of claim 1, wherein the compound represented by Chemical Formula 1 is represented by any one of the following Chemical Formulae 1-1 to 1-4:

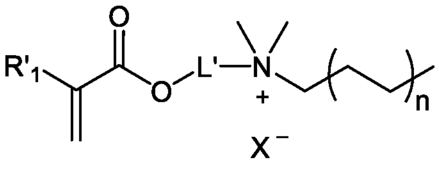

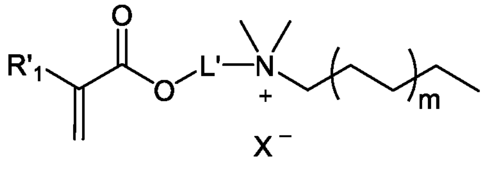

1-1

1-2

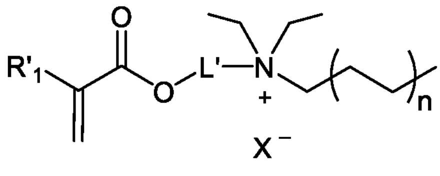

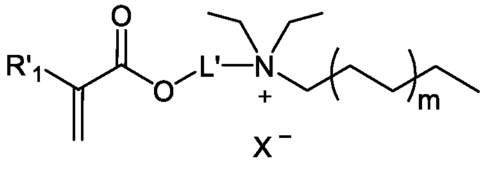

1-3

1-4

in Chemical Formulae 1-1 to 1-4,

R'$_1$ is hydrogen or methyl,
L' is methylene or ethylene,
$X^-$ is the same as defined in Claim 1,
n is an integer of 2 to 7, and
m is an integer of 2 to 6.

**11.** The compound of claim 1,
wherein the compound is any one selected from the group consisting of the following compounds:

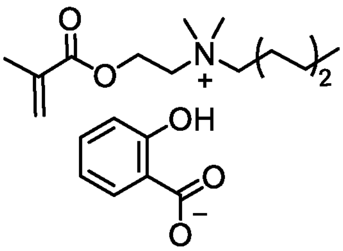

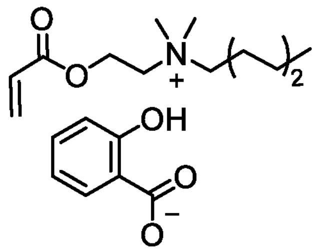

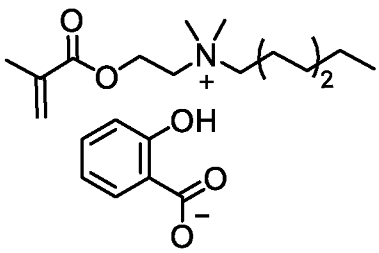

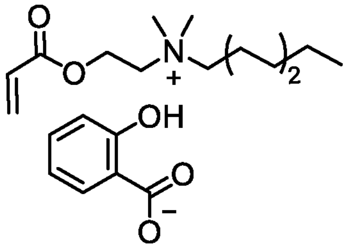

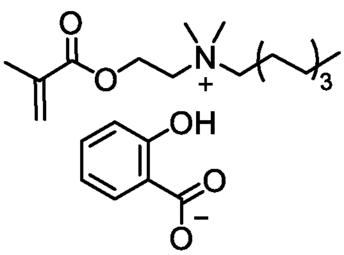

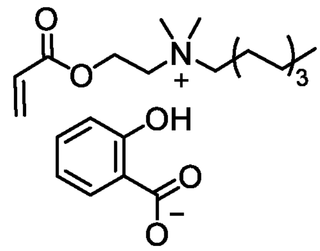

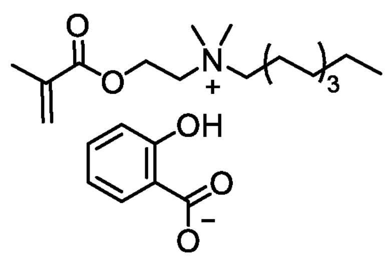
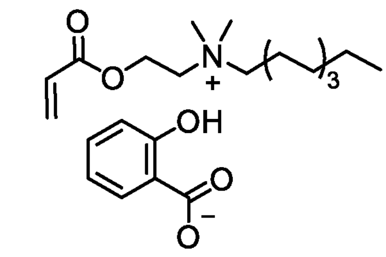

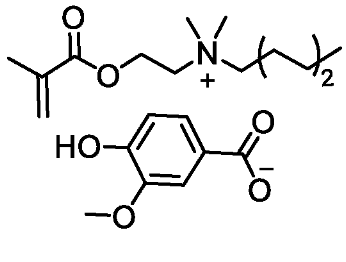
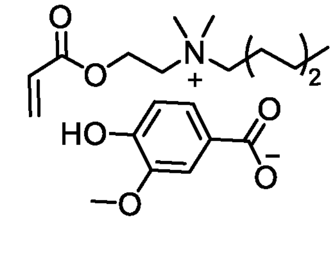

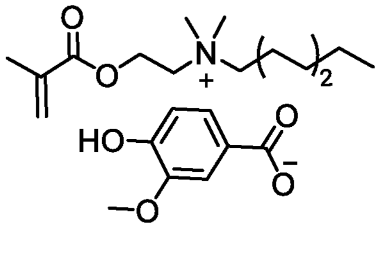
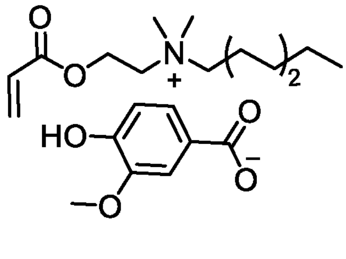

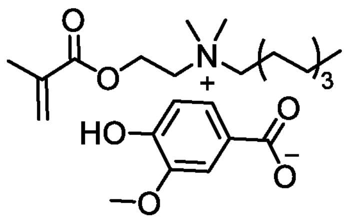
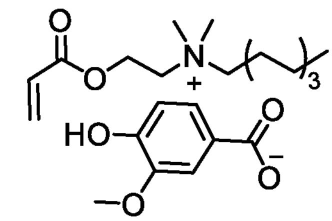

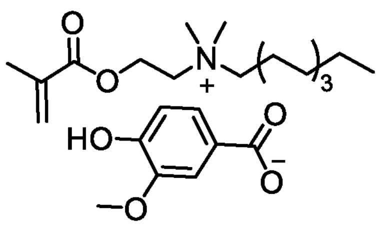
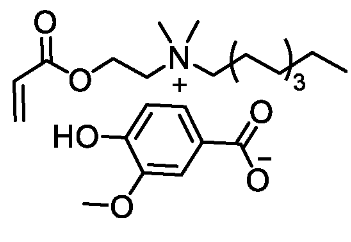

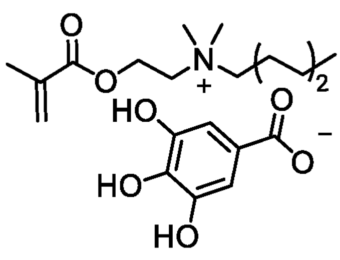
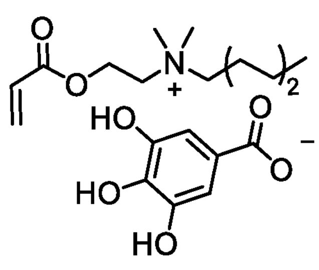

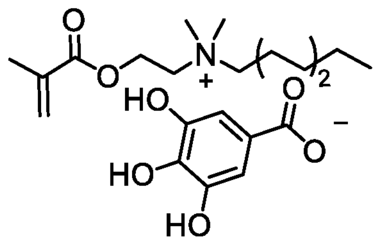

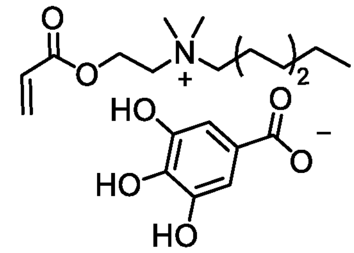

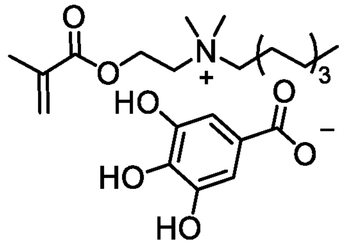

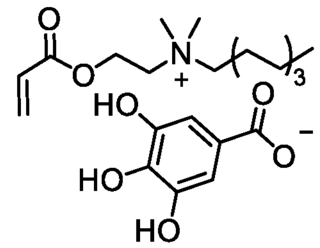

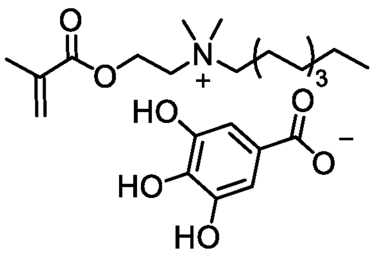

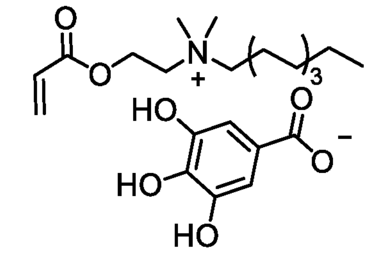

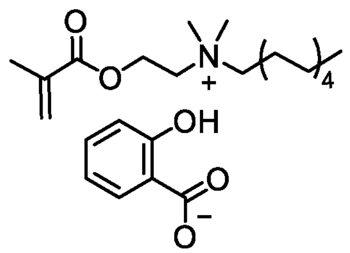

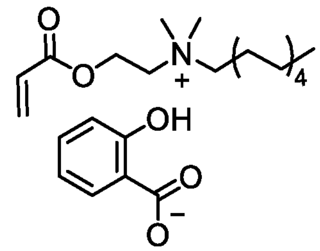

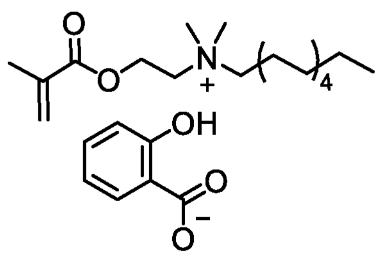

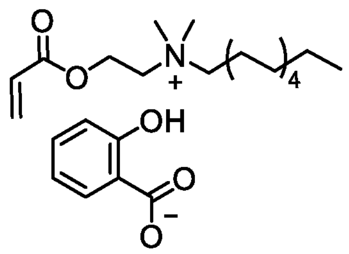

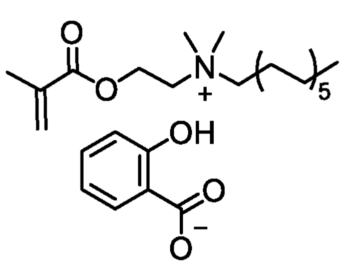

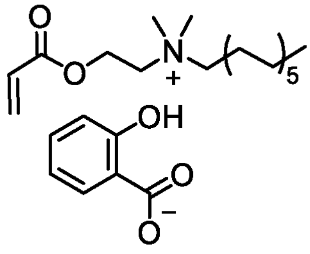

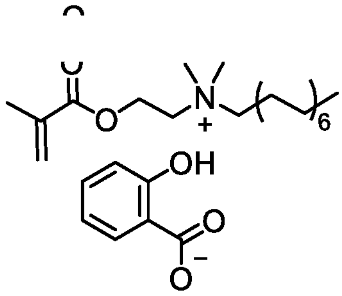

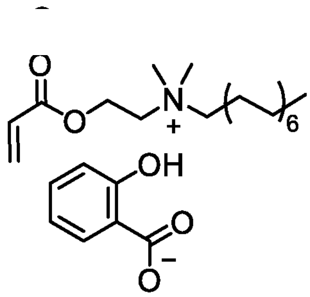

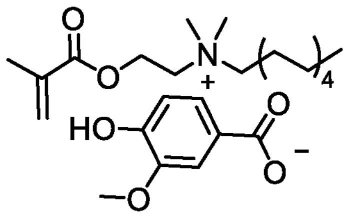

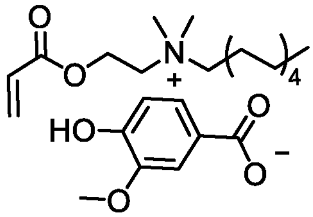

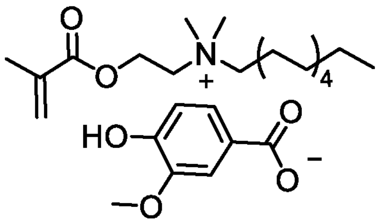

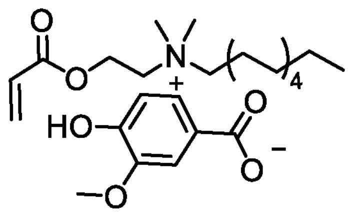

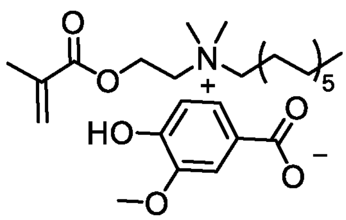

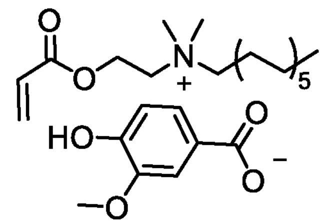

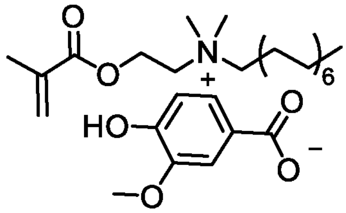

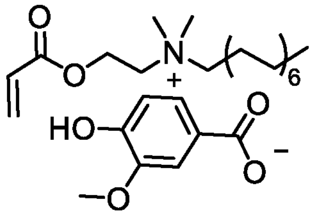

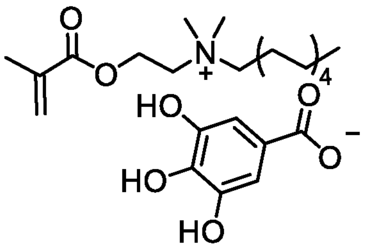

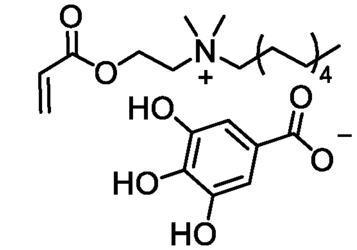

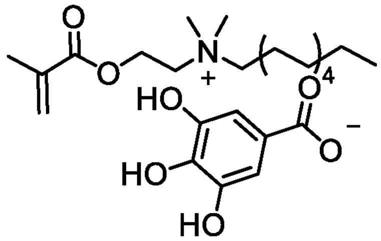

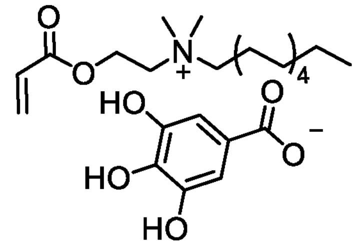

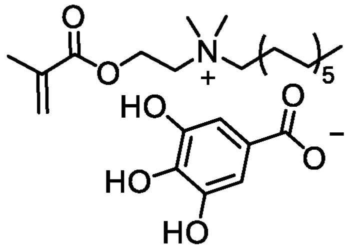

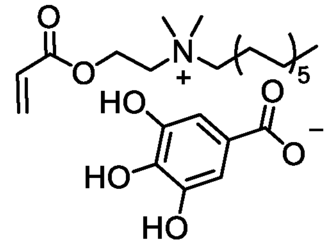

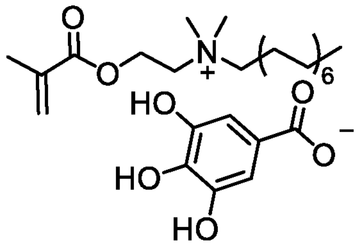

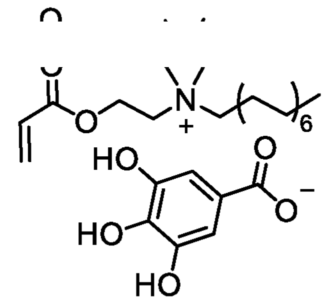

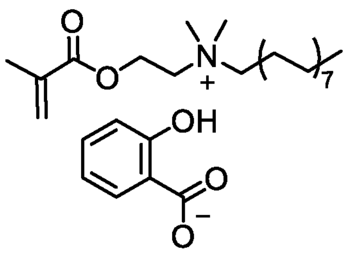

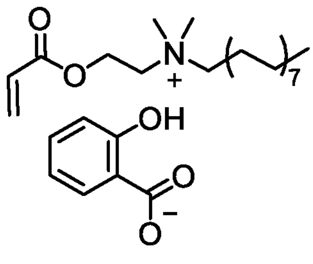

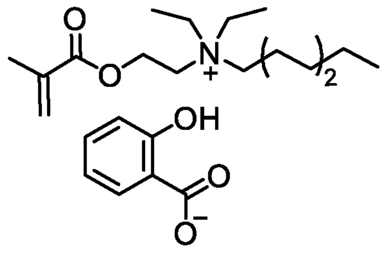

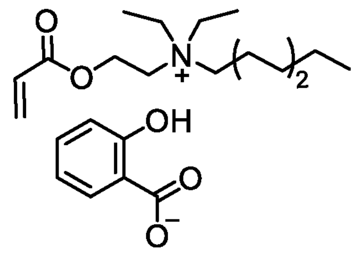

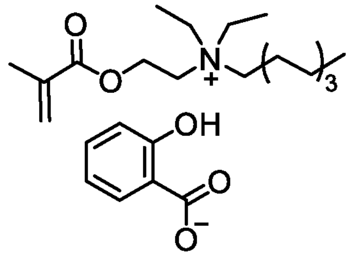

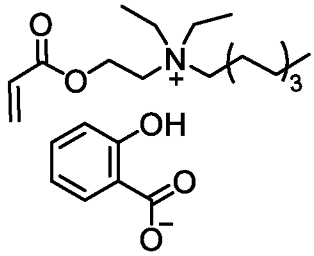

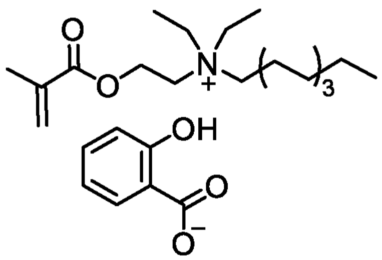

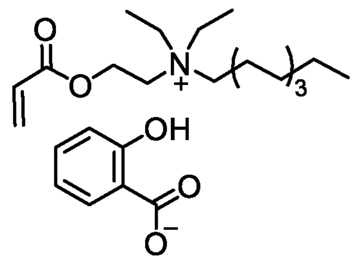

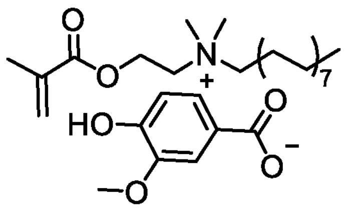

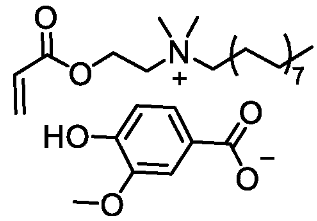

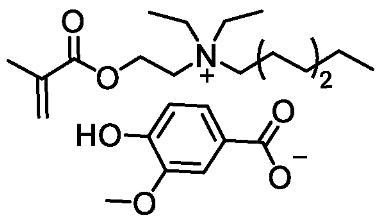

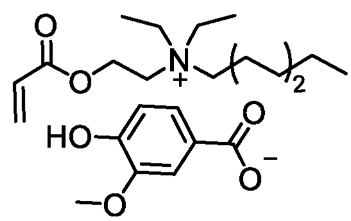

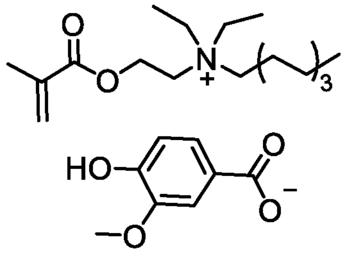

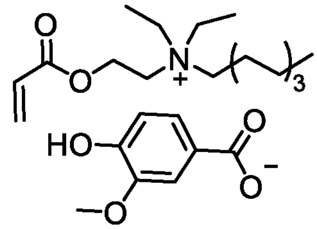

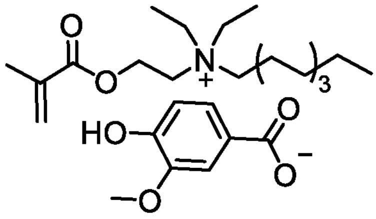

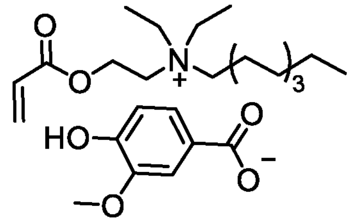

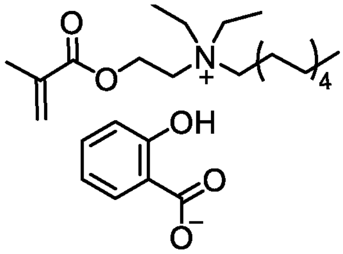

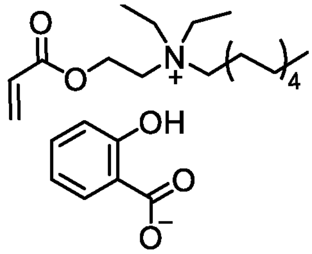

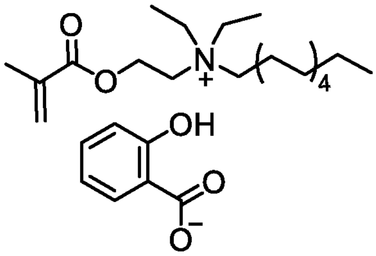

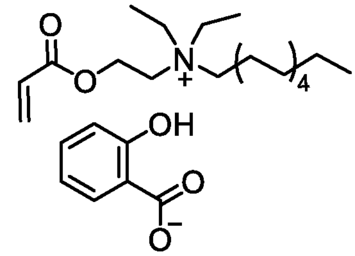

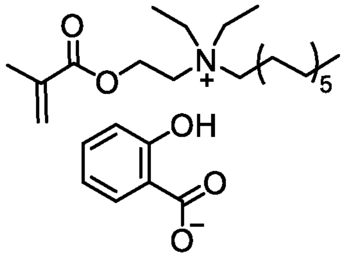

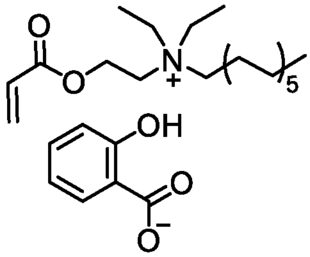

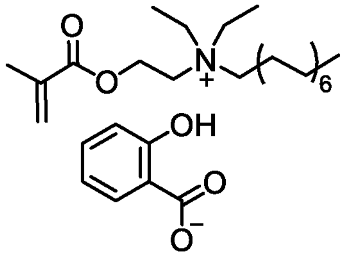

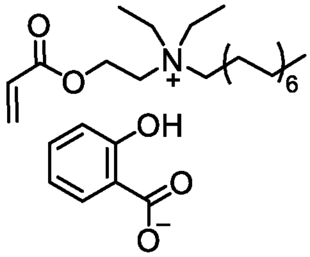

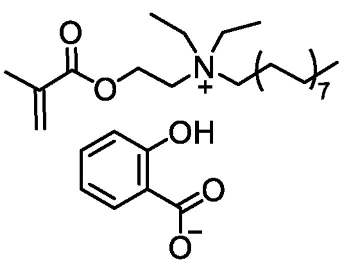

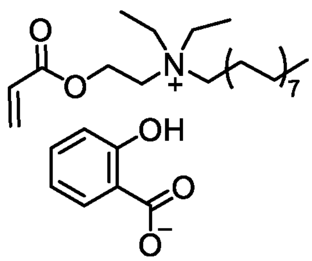

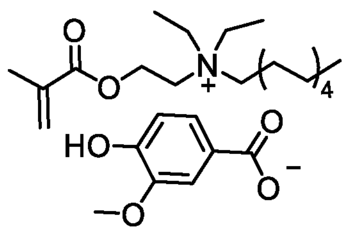

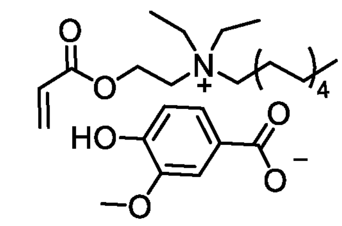

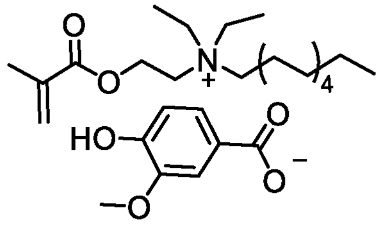

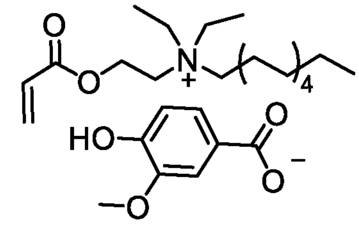

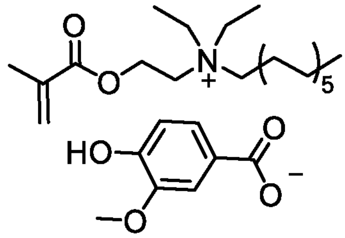

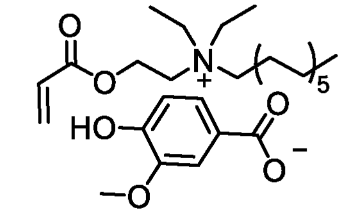

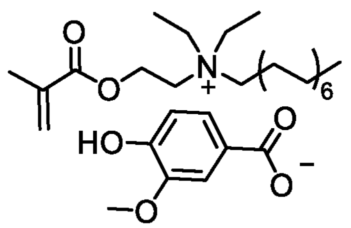

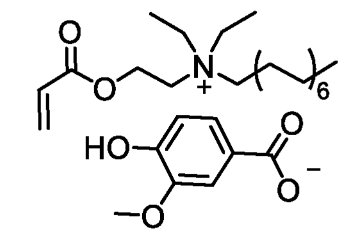

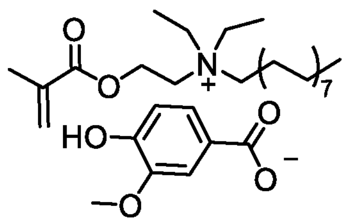

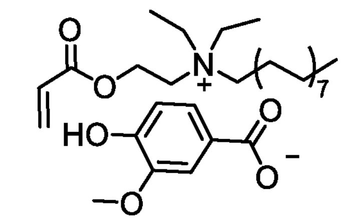

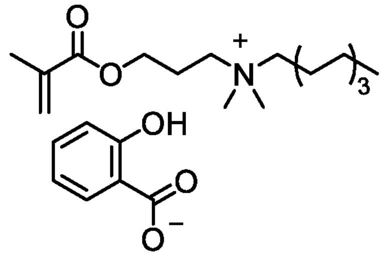

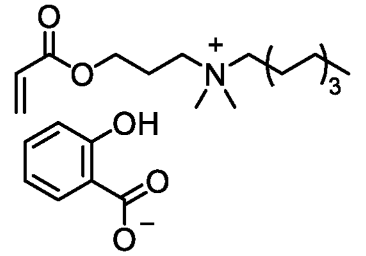

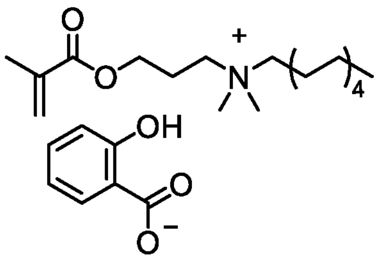

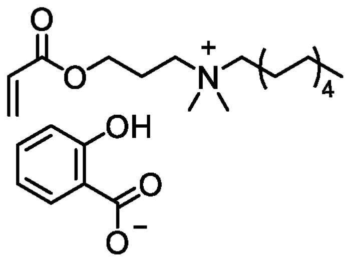

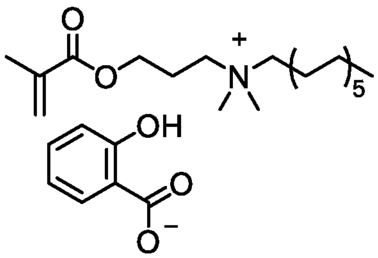

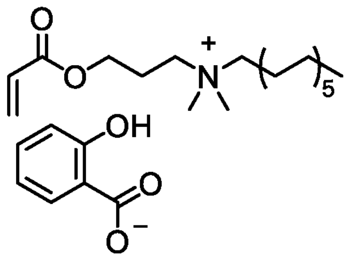

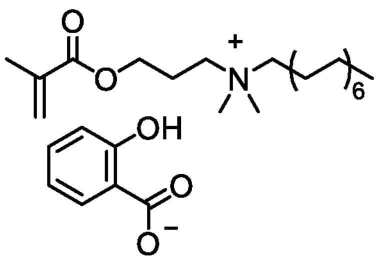

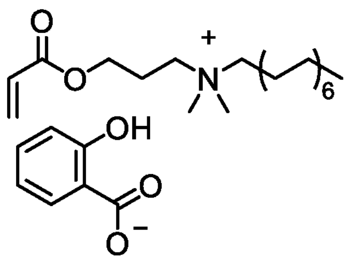

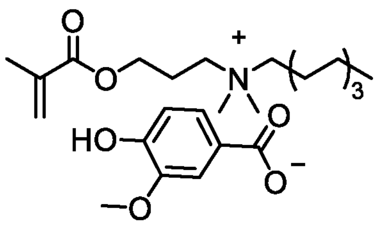

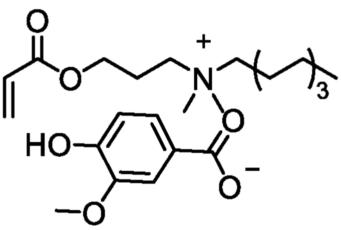

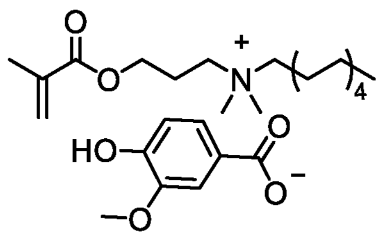

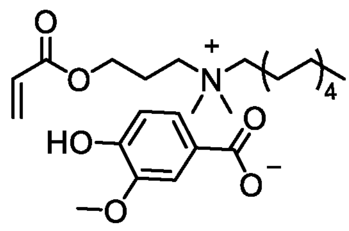

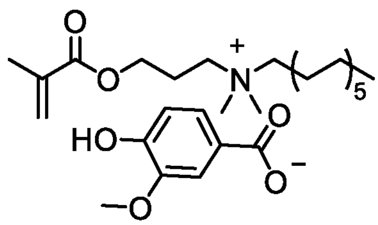

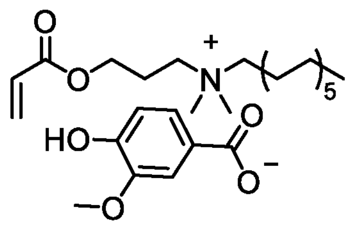

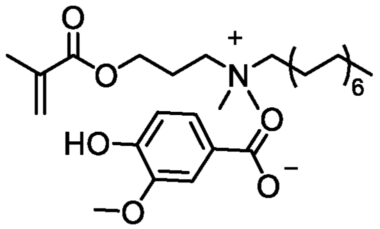

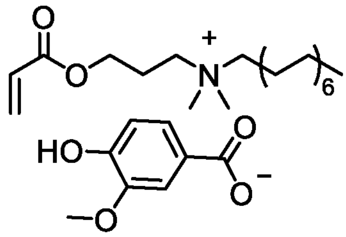

.

12. The compound of claim 1,
wherein the compound exhibits an antibacterial property against one or more of a Gram-positive bacterium and a Gram-negative bacterium.

13. The compound of claim 12,

wherein the Gram-negative bacterium is *Proteus mirabilis* or *Escherichia coli,* and
the Gram-positive bacterium is *Enterococcus faecalis.*

14. The compound of claim 12,
wherein the compound exhibits the antibacterial property against both of Gram-positive bacterium and Gram-negative bacterium.

15. An antibacterial agent comprising the compound of any one of claims 1 to 14.

【FIG. 1】

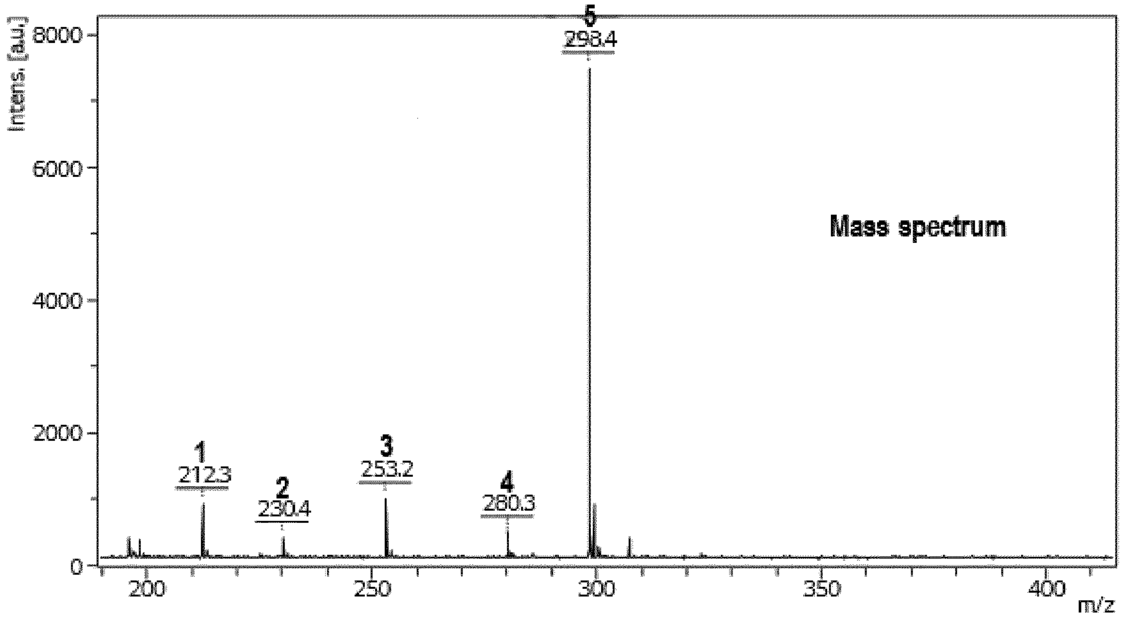
Intens. [a.u.]
8000
6000
4000
2000
0
Mass spectrum
1
212.3
2
230.4
3
253.2
4
280.3
5
298.4
200
250
300
350
400
m/z

【FIG. 2】

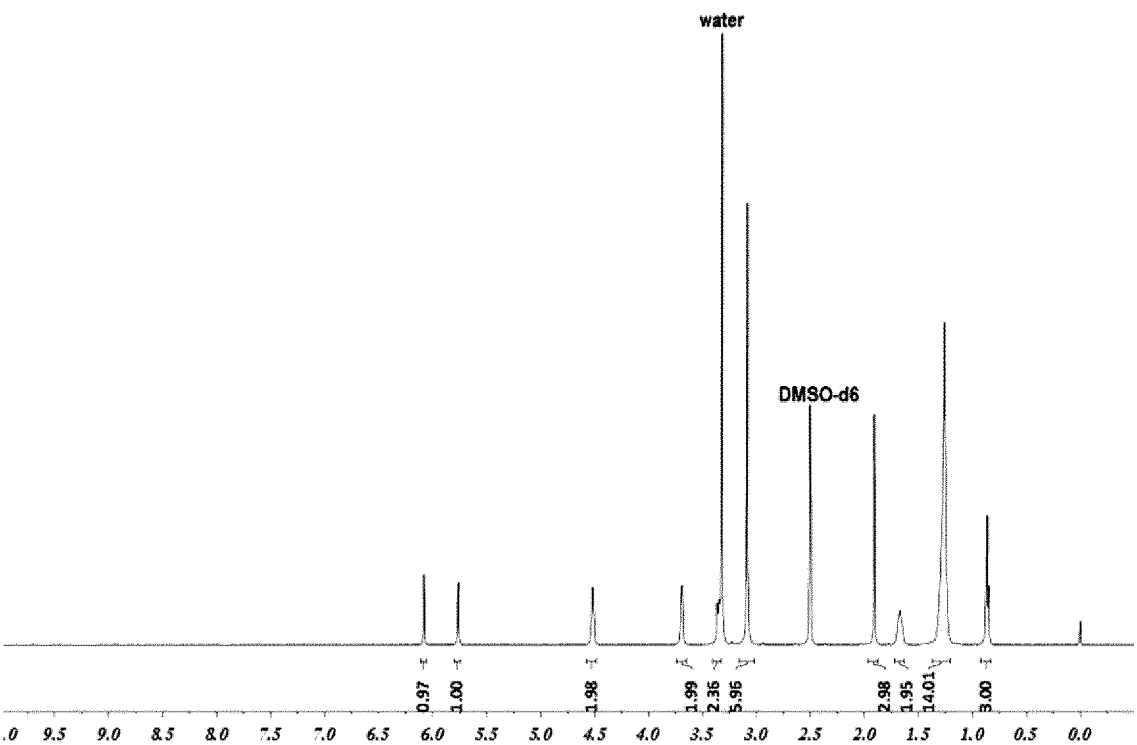
water
DMSO-d6
0.97
1.00
1.98
1.99
2.36
5.96
2.98
1.95
14.01
3.00
.0
9.5
9.0
8.5
8.0
7.5
7.0
6.5
6.0
5.5
5.0
4.5
4.0
3.5
3.0
2.5
2.0
1.5
1.0
0.5
0.0

【FIG. 3】

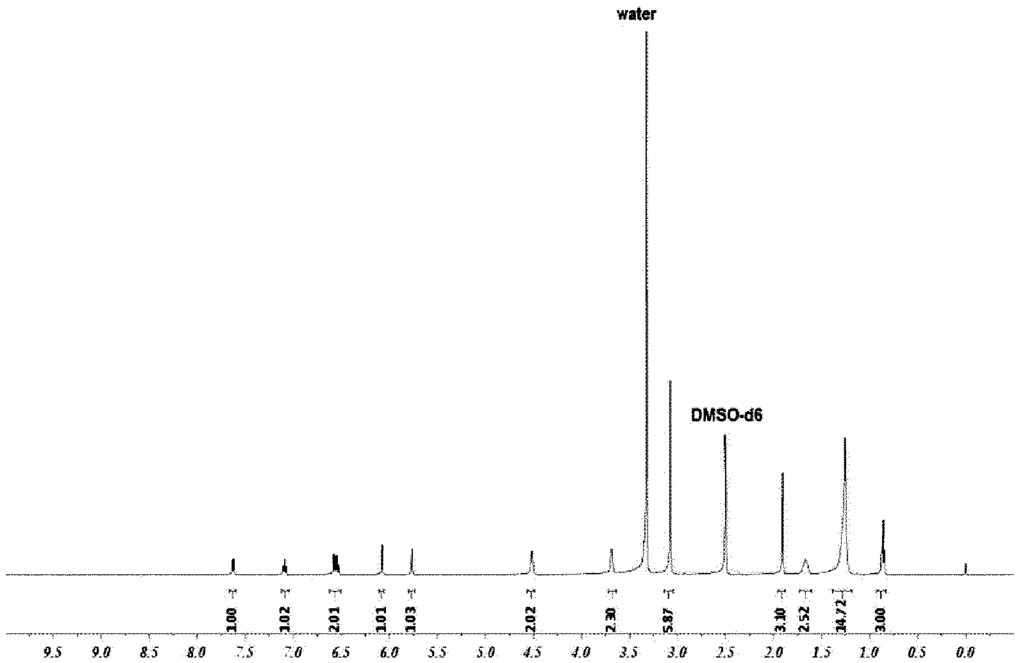
water
DMSO-d6
1.00
1.02
2.01
1.01
1.03
2.02
2.30
5.87
3.10
2.52
34.72
3.00
9.5 9.0 8.5 8.0 7.5 7.0 6.5 6.0 5.5 5.0 4.5 4.0 3.5 3.0 2.5 2.0 1.5 1.0 0.5 0.0

【FIG. 4】

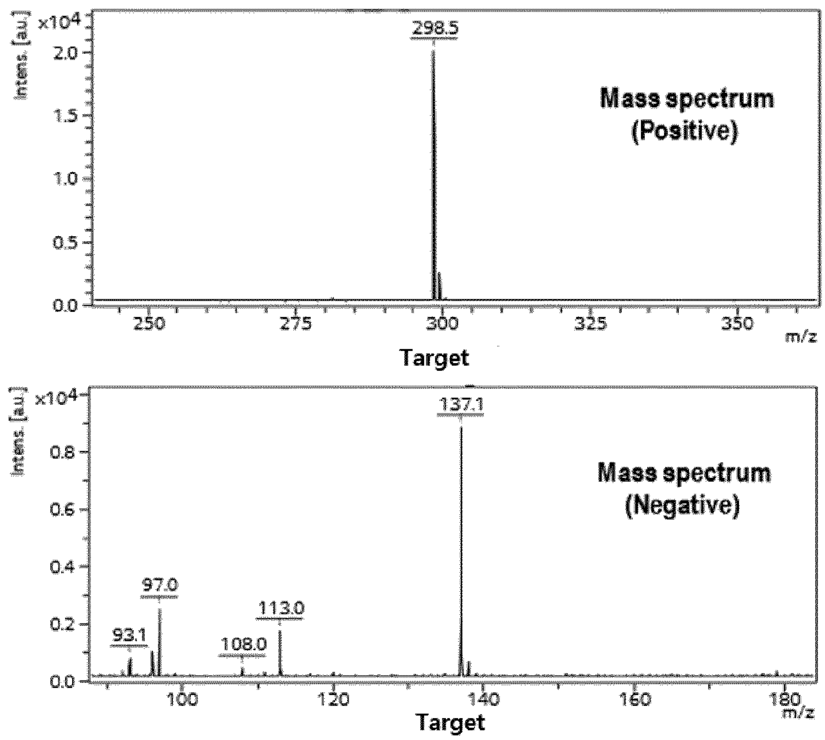
Intens. [a.u.]
x10⁴
2.0
1.5
1.0
0.5
0.0
298.5
Mass spectrum
(Positive)
250
275
300
325
350
m/z
Target
Intens. [a.u.]
x10⁴
0.8
0.6
0.4
0.2
0.0
137.1
97.0
93.1
108.0
113.0
Mass spectrum
(Negative)
100
120
140
160
180
m/z
Target

**INTERNATIONAL SEARCH REPORT**

International application No. **PCT/KR2022/009225**

**A. CLASSIFICATION OF SUBJECT MATTER**

**C07C 219/06**(2006.01)i; **C07C 219/20**(2006.01)i; **A01N 37/44**(2006.01)i

According to International Patent Classification (IPC) or to both national classification and IPC

**B. FIELDS SEARCHED**

Minimum documentation searched (classification system followed by classification symbols)

C07C 219/06(2006.01); A01N 25/12(2006.01); A01N 37/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 항균제(anti-bacterial agent, anti-microbial agent), 4차 암모늄 양이온(quaternary ammonium counter ion), 살리실레이트(salicylate)

**C. DOCUMENTS CONSIDERED TO BE RELEVANT**

| Category* | Citation of document, with indication, where appropriate, of the relevant passages | Relevant to claim No. |
|---|---|---|
| X<br>Y | US 2011-0195104 A1 (JIANG, S. et al.) 11 August 2011 (2011-08-11)<br>See claims 1-10; paragraphs [0296] and [0297]; and figure 13. | 1-4,7-9,12-15<br>5,6,10,11 |
| Y | FARAH, S. et al. Quaternary ammonium poly(diethylaminoethyl methacrylate)possessing antimicrobial activity. Colloids and surfaces B: Biointerfaces. 2015, vol. 128, pp. 608-613.<br>See abstract; formula 1; and figure 2. | 5,6,10,11 |
| Y | WANG, C.-G. et al. Self-catalyzed living radical polymerization using quaternary-ammonium-iodide-containing monomers. Macromolecules. 2019, vol. 52, no. 5, pp. 2712-2718.<br>See abstract; pages 2712-2714 and 2716; figures 1 and 2; and table 1; entry 3. | 5,6,10,11 |
| Y | ZHAO, J. et al. Self-stratified antimicrobial acrylic coatings via one-step UV curing. ACS applied materials & interfaces. 2015, vol. 7, no. 33, pp. 18467-18472.<br>See abstract; and formula 1. | 5,6,10,11 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **06 October 2022** | **06 October 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

INTERNATIONAL SEARCH REPORT

International application No.
PCT/KR2022/009225

C. DOCUMENTS CONSIDERED TO BE RELEVANT

| Category* | Citation of document, with indication, where appropriate, of the relevant passages | Relevant to claim No. |
|---|---|---|
| Y | FU, Y. et al. Robust liquid-repellent coatings based on polymer nanoparticles with excellent self-cleaning and antibacterial performances. Journal of materials chemistry A: Materials for energy and sustainability. 2017, vol. 5, no. 1, pp. 275-284. See abstract; pages 275-279; and formula 1. | 5,6,10,11 |

Form PCT/ISA/210 (second sheet) (July 2019)

**INTERNATIONAL SEARCH REPORT**
**Information on patent family members**

International application No.
**PCT/KR2022/009225**

| Patent document cited in search report | Publication date (day/month/year) | Patent family member(s) | Publication date (day/month/year) |
|---|---|---|---|
| US 2011-0195104 A1 | 11 August 2011 | AU 2008-326438 A1 | 28 May 2009 |
| | | AU 2008-326438 B2 | 04 September 2014 |
| | | CA 2705485 A1 | 28 May 2009 |
| | | CN 101918462 A | 15 December 2010 |
| | | CN 101918462 B | 17 July 2013 |
| | | EP 2225331 A2 | 08 September 2010 |
| | | EP 2225331 B1 | 06 January 2016 |
| | | EP 2227496 A1 | 15 September 2010 |
| | | EP 2227496 B1 | 29 October 2014 |
| | | JP 2011-503332 A | 27 January 2011 |
| | | JP 2011-504526 A | 10 February 2011 |
| | | JP 2014-012861 A | 23 January 2014 |
| | | JP 2015-083699 A | 30 April 2015 |
| | | JP 5474808 B2 | 16 April 2014 |
| | | US 10544312 B2 | 28 January 2020 |
| | | US 2009-0156460 A1 | 18 June 2009 |
| | | US 2010-0247614 A1 | 30 September 2010 |
| | | US 2010-0249267 A1 | 30 September 2010 |
| | | US 2013-0011363 A1 | 10 January 2013 |
| | | US 2013-0178125 A1 | 11 July 2013 |
| | | US 2017-0174907 A1 | 22 June 2017 |
| | | US 8268301 B2 | 18 September 2012 |
| | | US 8349966 B2 | 08 January 2013 |
| | | US 8404224 B2 | 26 March 2013 |
| | | US 8658192 B2 | 25 February 2014 |
| | | US 9533006 B2 | 03 January 2017 |
| | | WO 2009-067562 A1 | 28 May 2009 |
| | | WO 2009-067565 A2 | 28 May 2009 |
| | | WO 2009-067565 A3 | 17 September 2009 |
| | | WO 2009-067566 A1 | 28 May 2009 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210093703
- KR 1020220078380
- KR 100601393